# EUROPÄISCHE PATENTANMELDUNG

(11) **EP 0 789 026 A1**
(43) Veröffentlichungstag der Anmeldung: **13.08.1997**
(21) Anmeldenummer: 97101510.2
(22) Anmeldetag: 31.01.1997
(51) Int. Cl.: C07D 413/14, C07D 413/04, A61K 31/42, A61K 31/44, A61K 31/47, A61K 31/495

(54) **Heteroaryl-Oxazolidinone und ihre Verwendung als antibakterielle Arzneimittel**

(30) Priorität: 06.02.1996 DE 19604244; 27.11.1996 DE 19649095
(71) Anmelder: BAYER AG, 51368 Leverkusen (DE)
(72) Erfinder: Riedl, Bernd, Dr., 42329 Wuppertal (DE); Häbich, Dieter, Dr., 42115 Wuppertal (DE); Stolle, Andreas, Dr., 42115 Wuppertal (DE); Ruppelt, Martin, Dr., 42329 Wuppertal (DE); Bartel, Stephan, Dr., 51465 Bergisch Gladbach (DE); Guarnieri, Walter, Dr., 53909 Zülpich (DE); Endermann, Rainer, Dr., 42113 Wuppertal (DE); Kroll, Hein-Peter, Dr., 42115 Wuppertal (DE)

(57) **Zusammenfassung**

Die Erfindung betrifft neue Heteroaryl-Oxazolidinone der allgemeinen Formel (I) 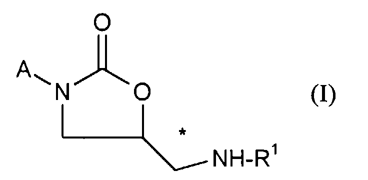 in welcher die Substituenten die in der Beschreibung angegebene Bedeutung haben, Verfahren zu ihrer Herstellung und ihre Verwendung als Arzneimittel, insbesondere als antibakterielle Arzneimittel.

## Beschreibung

Die vorliegende Erfindung betrifft neue Heteroaryl-Oxazolidinone, Verfahren zu ihrer Herstellung und ihre Verwendung als Arzneimittel, insbesondere als antibakterielle Arzneimittel.

Aus den Publikationen US 5 254 577, US 4 705 799, EP 311 090, EP 312 000 und C.H. Park et al., J. Med. Chem. 35, 1156 (1992) sind N-Aryloxazolidinone mit antibakterieller Wirkung bekannt. Außerdem sind 3-(Stickstoff-substituierte)-phenyl-5-beta-amidomethyloxazolidin-2-one aus der EP 609 905 A1 bekannt.

Ferner sind in der EP 609 441 und EP 657 440 Oxazolidinonderivate mit einer Monoaminoxidase inhibitorischen Wirkung und in der EP 645 376 mit Wirkung als Adhäsionsrezeptor-Antagonisten publiziert.

Antibakteriell wirksame Oxazolidinonderivate sind auch in unseren Anmeldungen EP 694 543, EP 693 491, EP 694 544 und EP 697 412 beschrieben.

Die vorliegende Erfindung betrifft neue Heteroaryl-Oxazolidinone der allgemeinen Formel (I) 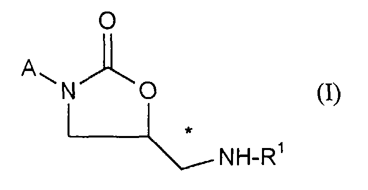 in welcher
- R¹: für einen Rest der Formel D-R², -CO-R³ oder -CO-NR⁴R⁵ steht,
worin
- D: die CO₂- oder SO₂-Gruppe bedeutet,
- R²: Phenyl oder geradkettiges oder verzweigtes Alkyl mit bis zu 7 Kohlenstoffatomen bedeutet,
- R³: Trifluormethyl oder geradkettiges oder verzweigtes Alkyl mit bis zu 6 Kohlenstoffatomen bedeutet, das durch Halogen oder Trifluormethyl substituiert ist,
- R⁴ und R⁵: gleich oder verschieden sind und
Wasserstoff, Phenyl oder geradkettiges oder verzweigtes Alkyl mit bis zu 5 Kohlenstoffatomen bedeuten,
- A: für einen über ein Kohlenstoffatom direkt gebundenen 6-gliedrigen, aromatischen Heterocyclus mit mindestens einem Stickstoffatom steht, oder für einen über ein Kohlenstoffatom direkt gebundenen, jeweils 6-gliedrigen, bi- oder tricyclischen aromatischen Rest mit mindestens einem stickstoffhaltigen Ring steht, oder
für β-Carbolin-3-yl oder für über den 6-Ring direkt gebundenes Indolizinyl steht, oder
für einen über ein Kohlenstoffatom direkt gebundenen 5-gliedrigen aromatischen Heterocyclus mit bis zu 3 Heteroatomen aus der Reihe S, N und/oder O steht, der zusätzlich einen annellierten Benzol- oder Naphthylring besitzen kann, wobei alle Cyclen gegebenenfalls jeweils bis zu 3-fach gleich oder verschieden durch Carboxy, Halogen, Cyano, Mercapto, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -NR⁶R⁷ substituiert sein kann,
worin
- R⁶ und R⁷: gleich oder verschieden sind und Wasserstoff, Cycloalkyl mit 3 bis 6 Kohlenstoffatomen oder geradkettiges oder verzweigtes Alkyl mit bis zu 5 Kohlenstoffatomen oder Phenyl bedeuten, oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,
und/oder
die Cyclen gegebenenfalls durch eine Gruppe der Formel -NR⁶'R⁷' substituiert sind,
worin
- R^{6'} und R^{7'}: gleich oder verschieden sind und die oben angegebene Bedeutung von R⁶ und R⁷ haben und mit diesen gleich oder verschieden sind,
und/oder
die Cyclen gegebenenfalls durch (C₂-C₈)-Alkenylphenyl, Phenyl oder durch einen 5- oder 6-gliedrigen gesättigten oder ungesättigten Heterocyclus mit bis zu 3 Heteroatomen aus der Reihe S, N und/oder O substituiert sind, die ihrerseits gegebenenfalls durch eine Gruppe der Formel -CO-NR⁸R⁹, -NR¹⁰R¹¹, -NR¹²-S(O)₂-R¹³, R¹⁴R¹⁵N-SO₂- oder R¹⁶-S(O)ₐ- substituiert sind,
worin
- a: eine Zahl 0, 1 oder 2 bedeutet,
- R⁸, R⁹, R¹², R¹⁴ und R¹⁵: gleich oder verschieden sind und Wasserstoff, geradkettiges oder verzweigtes Alkyl mit bis zu 6 Kohlenstoffatomen oder Phenyl bedeuten,
- R¹⁰ und R¹¹: gleich oder verschieden sind und die oben angegebene Bedeutung von R⁶ und R⁷ haben und mit diesen gleich oder verschieden sind,
- R¹³ und R¹⁶: gleich oder verschieden sind und
geradkettiges oder verzweigtes Alkyl mit bis zu 4 Kohlenstoffatomen oder Phenyl bedeuten, das gegebenenfalls durch geradkettiges oder verzweigtes Alkyl mit bis zu 4 Kohlenstoffatomen substituiert ist,
und/oder ihrerseits gegebenenfalls bis zu 2-fach gleich oder verschieden durch Carboxy, Halogen, Cyano, Mercapto, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -NR¹⁷R¹⁸ substituiert sein kann,
worin
- R¹⁷ und R¹⁸: die oben angegebene Bedeutung von R⁶ und R⁷ haben und mit diesen gleich oder verschieden sind,
und/oder
die Cyclen gegebenenfalls durch einen Rest der Formel 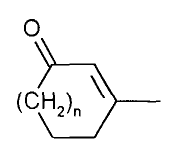 substituiert sind
worin
- n: eine Zahl 0, 1 oder 2 bedeutet
und deren Salze.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomerer einheitlichen Bestandteile trennen.

Physiologisch unbedenkliche Salze der neuen Heteroaryl-Oxazolidinone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methyl-piperidin.

Heterocyclus unter dem Substituenten A im Fall der direkten Anbindung an das Oxazolidinongerüst kann im Rahmen der Erfindung zum einen für einen 5-gliedrigen aromatischen Ring stehen, der als Heteroatome bis zu 3 Sauerstoff-, Schwefel- und/oder Stickstoffatome enthalten und zusätzlich einen annellierten Benzol- oder Naphthylring besitzen kann. Beispielsweise werden genannt: Pyrrolyl, Imidazolyl, Furyl, Thienyl, Thiazolyl, Oxazolyl, Isothiazolyl, Isoxazolyl, Furazanyl, Indolyl, Benzo[b]thienyl, Naphtho[2,3-b]thienyl, Benzo[b]thiazolyl, Benzo[b]furanyl oder Benzo[b]imidazolyl. Bevorzugt sind Pyrrolyl, Imidazolyl, Furyl, Thienyl, Isothiazolyl, Thiazolyl, Isoxazolyl, Furazanyl, Oxazolyl, Benzo[b]thienyl, Benzo-[b]imidazolyl und Benzo[b]thiazolyl.

Weiterhin kann Heterocyclus unter dem Substituenten A im Fall der direkten Anbindung an das Oxazolidinongerüst in Rahmen der Erfindung im allgemeinen auch für einen über ein Kohlenstoffatom direkt gebundenen 6-gliedrigen, aromatischen Heterocyclus mit mindestens einem Stickstoffatom, oder für einen über ein Kohlenstoffatom, direkt gebundenem jeweils 6-gliedrigen, bi- oder tricyclischen aromatischen Rest mit mindestens einem stickstoffhaltigem Ring, oder für β-Carbolin-3-yl oder für über den 6-Ring direkt gebundenes Indolizinyl stehen Beispielsweise seien genannt Cinnolinyl, Pteridinyl, Phenanthridinyl, Acridinyl, Phenanthrolinyl, Chinazolinyl, Chinoxalinyl, Naphthyridinyl, Phthalazinyl, Chinolyl, Isochinolyl, 4H-Chinolizinyl, Phenazinyl, Pyridyl, Pyrazinyl, Pyrimidinyl, Pyridazinyl, Indolizinyl, β-Carbolin-3-yl und über den 6-Ring direkt gebundenes Indolizinlyl.

Im weiteren Substitutionsfeld steht Heterocyclus auch für einen 5- bis 6-gliedrigen, gesättigten oder ungesättigten Ring, der als Heteroatome bis zu 3 Sauerstoff-, Schwefel- und/oder Stickstoffatome enthalten kann. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl oder Piperazinyl.

Dazu gehören auch über N gebundene, 5- bis 6-gliedrige gesättigte Heterocyclen, die außerdem als Heteroatome bis zu 2 Sauerstoff-, Schwefel- und/oder Stickstoffatome enthalten können, wie beispielsweise Piperidyl, Morpholinyl oder Piperazinyl oder Pyrrolidinyl. Besonders bevorzugt sind Piperidyl und Pyrrolidinyl.

Hydroxyschutzgruppe im Rahmen der oben angegebenen Definition steht im allgemeinen für eine Schutzgruppe aus der Reihe; Trimethylsilyl, Triisopropylsilyl, tert.Butyl-dimethylsilyl, Benzyl, Benzyloxycarbonyl, 2-Nitrobenzyl, 4-Nitrobenzyl, tert. Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, tert. Butyldimethylsilyl oder Tetrahydropyranyl.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl.

Bevorzugt sind Verbindungen der allgemeinen Formel (I),
in welcher
- R¹: für einen Rest der Formel D-R², -CO-R³ oder -CO-NR⁴R⁵ steht,
worin
- D: die CO₂- oder SO₂-Gruppe bedeutet,
- R²: Phenyl oder geradkettiges oder verzweigtes Alkyl mit bis zu 5 Kohlenstoffatomen bedeutet,
- R³: Trifluormethyl oder geradkettiges oder verzweigtes Alkyl mit bis zu 5 Kohlenstoffatomen bedeutet, das durch Fluor, Chlor, Brom oder Trifluormethyl substituiert ist,
- R⁴ und R⁵: gleich oder verschieden sind und
Wasserstoff, Phenyl oder geradkettiges oder verzweigtes Alkyl mit bis zu 4 Kohlenstoffatomen bedeuten,
- A: für über ein Kohlenstoffatom gebundenes Cinnolinyl, Pteridinyl, Acridinyl, Chinazolinyl, Chinoxalinyl, Naphthyridinyl, Phthalazinyl, Chinolyl, Isochinolyl, Pyridyl, Pyrazinyl, Pyrimidinyl oder Pyridazinyl steht, oder für über ein Kohlenstoffatom direkt gebundenes Pyrrolyl, Imidazolyl, Furyl, Thienyl, Thiazolyl, Oxazolyl, Isothiazolyl, Isoxazolyl oder Furazanyl steht, oder für ebenfalls über ein Kohlenstoffatom des 5-gliedrigen Ringes direkt gebundenes Indolyl, Benzo[b]thienyl, Naphto[2,3-b]thienyl, Benzo[b]thiazolyl, Benzo[b]imidazolyl oder Benzo[b]furanyl steht, die gegebenenfalls bis zu 3-fach gleich oder verschieden durch Fluor, Chlor, Brom, geradkettiges oder verzweigtes Alkyl, Acyl oder Alkoxy mit jeweils bis zu 5 Kohlenstoffatomen oder durch Phenyl, Pyrimidyl, Pyridazinyl oder Pyridyl substituiert sind, die ihrerseits durch geradkettiges oder verzweigtes Alkyl, Alkoxy oder Acyl mit jeweils bis zu 5 Kohlenstoffatomen, oder durch eine Gruppe der Formel -NR⁶R⁷ substituiert sein können,
worin
- R⁶ und R⁷: gleich oder verschieden sind und
Wasserstoff, Phenyl, Cyclopropyl, Cyclobutyl, Cyclopentyl, Cyclohexyl oder geradkettiges oder verzweigtes Alkyl mit bis zu 4 Kohlenstoffatomen bedeuten,
und deren Salze.

Besonders bevorzugt sind erfindungsgemäße Verbindungen der allgemeinen Formel (I),
in welcher
- R¹: für einen Rest der Formel D-R², -CO-R³ oder -CO-NR⁴R⁵ steht,
worin
- D: die CO₂- oder SO₂-Gruppe bedeutet,
- R²: Phenyl oder geradkettiges oder verzweigtes Alkyl mit bis zu 4 Kohlenstoffatomen bedeutet,
- R³: Trifluormethyl oder geradkettiges oder verzweigtes Alkyl mit bis zu 3 Kohlenstoffatomen bedeutet, das durch Fluor, Chlor, Brom oder Trifluormethyl substituiert ist,
- R⁴ und R⁵: gleich oder verschieden sind und
Wasserstoff Phenyl oder geradkettiges oder verzweigtes Alkyl mit bis zu 3 Kohlenstoffatomen bedeuten,
- A: für Thienyl, Pyridyl oder Chinolyl steht, die gegebenenfalls bis zu 2-fach gleich oder verschieden durch Chlor, Brom, geradkettiges oder verzweigtes Alkyl oder Alkoxy mit jeweils bis zu 5 Kohlenstoffatomen oder durch Phenyl, Pyridazinyl, Pyrimidyl oder Pyridyl substituiert sind, die ihrerseits durch geradkettiges oder verzweigtes Alkyl, Alkoxy oder Acyl mit jeweils bis zu 5 Kohlenstoffatomen, oder durch eine Gruppe der Formel -NR⁶R⁷ substituiert sein können,
worin
- R⁶ und R⁷: gleich oder verschieden sind und
Wasserstoff; Phenyl, Cyclopropyl, Cyclobutyl, Cyclopentyl, Cyclohexyl oder geradkettiges oder verzweigtes Alkyl mit bis zu 3 Kohlenstoffatomen bedeuten,
und deren Salze.

Weiterhin besonders bevorzugt sind erfindungsgemäße Verbindungen der allgemeinen Formel (I), in welcher
- R¹: für einen Rest der Formel D-R² steht,
worin
- D: die SO₂-Gruppe bedeutet und
- R²: wie oben angegeben definiert ist.

Weiterhin besonders bevorzugt sind erfindungsgemäße Verbindungen der allgemeinen Formel (I), in welcher
- R¹: für einen Rest der Formel D-R² steht,
worin
- D: die CO₂-Gruppe bedeutet und
- R²: wie oben angegeben definiert ist.

Weiterhin besonders bevorzugt sind erfindungsgemäße Verbindungen der allgemeinen Formel (I), in welcher
- R¹: für einen Rest der Formel CO-R³ steht,
worin
- R³: wie oben angegeben definiert ist.

Weiterhin besonders bevorzugt sind erfindungsgemäße Verbindungen der allgemeinen Formel (I), in welcher
- R¹: für einen Rest der Formel CO-NR⁴R⁵ steht,
worin
- R⁴ und R⁵: wie oben angegeben definiert sind.

Weiterhin besonders bevorzugt sind Verbindungen der allgemeinen Formel (I), in welcher
- A: für Thienyl steht, das gegebenenfalls wie oben angegeben substituiert sein kann.

Weiterhin besonders bevorzugt sind Verbindungen der allgemeinen Formel (I), in welcher
- A: für Chinolyl steht, das gegebenenfalls wie oben angegeben substituiert sein kann.

Weiterhin besonders bevorzugt sind Verbindungen der allgemeinen Formel (I), in welcher
- A: für Pyridyl steht, das gegebenenfalls wie oben angegeben substituiert sein kann Von diesen Verbindungen ganz besonders bevorzugt sind beispielsweise solche, in denen das Pyridyl durch Pyrimidyl oder Pyridyl substituiert ist, die ihrerseits durch geradkettiges oder verzweigtes Alkyl oder Alkoxy mit jeweils bis zu 5 Kohlenstoffatomen substituiert sein können

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II) 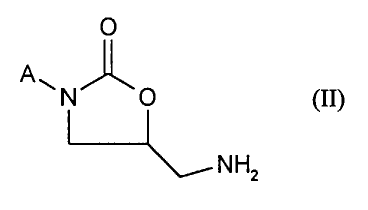 in welcher
- A: die oben angegebene Bedeutung hat,
mit Verbindungen der allgemeinen Formel (III)

R¹-E (III)

in welcher
- R¹: die oben angegebene Bedeutung hat,
und
- E: für Halogen oder für geradkettiges oder verzweigtes Alkylthio, Alkoxy oder Oxyalkoxycarbonyl mit jeweils bis zu 5 Kohlenstoffatomen steht,
in inerten Lösemitteln, gegebenenfalls in Anwesenheit einer Base umsetzt.

Das erfindungsgemäße Verfahren kann durch folgendes Formelschema beispielhaft erläutert werden:

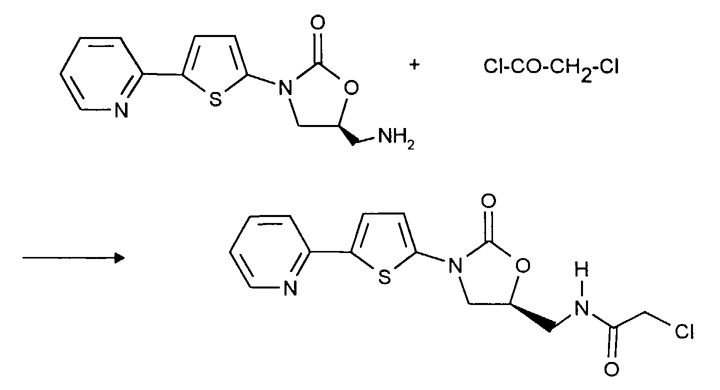

Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylamide, wie beispielsweise Lithium-N-(bis)triphenylsilylamid oder Lithiumalkyle wie n-Butyllithium.

Die Base wird in einer Menge von 1 mol bis 10 mol, bevorzugt von 1 mol bis 3 mol, bezogen auf 1 mol der Verbindungen der allgemeinen Formel (III) eingesetzt.

Die Umsetzung wird im allgemeinen bei normalem, erhöhtem oder bei erniedrigtem Druck durchgeführt (z.B. 0,5 bis 5 bar). Im allgemeinen arbeitet man bei Normaldruck.

Die Verbindungen der allgemeinen Formel (III) sind bekannt oder nach üblichen Methoden herstellbar.

Die Verbindungen der allgemeinen Formel (II) sind teilweise neu und können hergestellt werden, indem man
Verbindungen der allgemeinen Formel (IV) 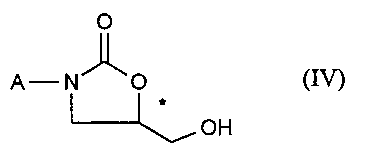 in welcher
- A: die oben angegebene Bedeutung hat,
durch Umsetzung mit (C₁-C₄)-Alkyl- oder Phenylsulfonsäurechloriden in inerten Lösemitteln und in Anwesenheit einer Base in die entsprechenden Verbindungen der allgemeinen Formel (V) 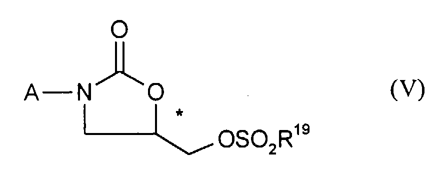 in welcher
- A: die oben angegebene Bedeutung hat
und
- R¹⁹: für C₁-C₄-Alkyl oder Phenyl steht,
überführt,
anschließend mit Natriumazid in inerten Lösemitteln die Azide der allgemeinen Formel (VI) 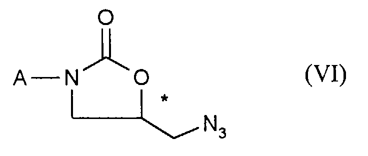 in welcher
- A: die oben angegebene Bedeutung hat,
herstellt,
in einem weiteren Schritt durch Umsetzung mit (C₁-C₄-Alkyl-O)₃-P oder PPh₃, vorzugsweise (CH₃O)₃P in inerten Lösemitteln und mit Säuren in die Amine überführt.

Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylamide, wie beispielsweise Lithium-N-(bis)triphenylsilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formel (IV) eingesetzt.

Alle Umsetzungen werden im allgemeinen bei normalem erhöhtem oder bei erniedrigtem Druck durchgeführt (z.B. 0,5 bis 5 bar). Im allgemeinen arbeitet man bei Normaldruck.

Die Reduktion der Azide erfolgt mit (CH₃O)₃P und Salzsäure.

Die Reduktion erfolgt im allgemeinen in einem Temperaturbereich von -50°C bis zum jeweiligen Siedepunkt des Lösemittels, bevorzugt von -20°C bis +90°C.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Die Verbindungen der allgemeinen Formeln (V) und (VI) sind neu und können wie oben beschrieben hergestellt werden.

Die Verbindungen der allgemeinen Formel (IV) sind teilweise neu und können hergestellt werden, indem man
[B] Verbindungen der allgemeinen Formeln (VII) oder (VIII)

A-N=C=O (VII)

oder

A-CO-N₃ (VIII)

in welchen
- A: die oben angegebenen Bedeutungen hat,
mit Lithiumbromid/(C₄H₉)₃ P(O) und Epoxiden der allgemeinen Formel (IX) 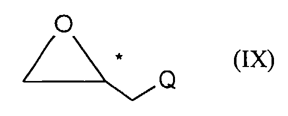 in welcher
- Q: für C₁-C₆-Acyloxy steht,
in inerten Lösemitteln, gegebenenfalls in Anwesenheit einer Base
umsetzt,
durch eine typische Esterverseifung oder durch eine typische Umesterung die Hydroxyfunktion freisetzt,
oder
[C] Verbindungen der allgemeinen Formel (X)

A-NH-CO₂-X (X)

in welcher
- A: die oben angegebene Bedeutung hat
und
- X: für eine typische Schutzgruppe, vorzugsweise Benzyl steht,
in inerten Lösemitteln und in Anwesenheit einer Base, beispielsweise Lithiumalkylen oder Lithium-N-alkyl- oder Lithium-N-silylalkylamiden, vorzugsweise n-Butyllithium, mit Epoxiden der allgemeinen Formel (IX) umsetzt,
oder
zunächst Verbindungen der allgemeinen Formel (VIII) durch Abspaltung von Stickstoff in Alkoholen in die Verbindungen der allgemeinen Formel (Xa)

A-NH-CO₂-Y (Xa)

in welcher
- A: die oben angegebene Bedeutung hat
und
- Y: für geradkettiges oder verzweigtes C₂-C₆-Alkyl, vorzugsweise n-Butyl steht,
überführt,
und in einem zweiten Schritt wie unter [B] beschrieben in inerten Lösemitteln und in Anwesenheit einer Base, vorzugsweise Lithium-N-alkyl- oder N-Silylalkylamiden oder n-Butyllithium mit Epoxiden der allgemeinen Formel (IX) umsetzt,
oder
[D] Verbindungen der allgemeinen Formel (XI) 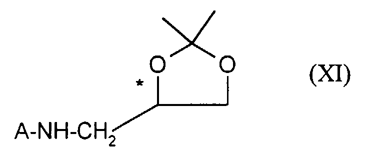 in welcher
- A: die oben angegebene Bedeutung hat,
entweder direkt mit Säuren und Kohlensäurediethylester
umsetzt,
oder zunächst durch Umsetzung der Verbindungen der allgemeinen Formel (XI) mit Säuren die Verbindungen der allgemeinen Formel (XII) 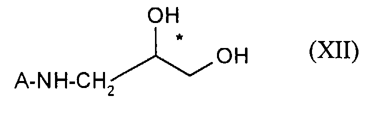 in welcher
- A: die oben angegebene Bedeutung hat,
herstellt, und anschließend in Anwesenheit eines Hilfsmittels in inerten Lösemitteln cyclisiert.

Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylamide, wie beispielsweise Lithium-N-(bis)triphenylsilylamid oder Lithiumalkyle wie n-Butyllithium.

Die Base wird in einer Menge von 1 mol bis 10 mol, bevorzugt von 1 mol bis 3 mol, bezogen auf 1 mol der Verbindungen der allgemeinen Formeln (IX) und (X), eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Das Verfahren [B] erfolgt bevorzugt in Xylol oder Dichlorbenzol, gegebenenfalls in Gegenwart von Triethylamin, unter Rückfluß.

Die basenkatalysierte Umesterung wird mit einem der oben aufgeführten Alkohole, vorzugsweise Methanol, in einem Temperaturbereich von -10°C bis +40°C, vorzugsweise bei Raumtemperatur durchgeführt.

Als Basen eignen sich im allgemeinen Natriumhydrogencarbonat, Natriummethanolat, Hydrazinhydrat, Kaliumcarbonat oder Caesiumcarbonat. Bevorzugt ist Caesiumcarbonat.

Das Verfahren [C] erfolgt in einem der oben aufgeführten Ether mit Lithium-alkylverbindungen oder Lithium-N-silylamiden, wie beispielsweise n-Butyllithium, Lithiumdiisopropylamid oder Lithium-bistrimethylsilylamid, vorzugsweise in Tetrahydrofuran mit Lithium-bis-trimethylsilylamid oder n-Butyllithium, in einem Temperaturbereich von -100°C bis +20°C, vorzugsweise von -75°C bis -40°C.

Für das Verfahren [D] eignen sich für den 1. Schritt vorzugsweise die oben aufgeführten Alkohole, im Falle der anschließenden Cyclisierung Tetrahydrofuran.

Als Basen für die Cyclisierung eignen sich vorzugsweise die oben aufgeführten Lithium-N-silylamidverbindungen oder n-Butyllithium. Besonders bevorzugt ist n-Butyllithium.

Der erste Reaktionsschritt wird bei der Siedetemperatur des entsprechenden Alkohols, die Cyclisierung in einem Temperaturbereich von -70°C bis Raumtemperatur durchgeführt.

Die Cyclisierung [D] wird in Anwesenheit eines Hilfsmittels und/oder Anwesenheit einer Säure durchgeführt.

Als Säuren eignen sich im allgemeinen anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure. Besonders bevorzugt ist Salzsäure.

Die Säure wird in einer Menge von 1 mol bis 10 mol, bevorzugt von 1 mol bis 2 mol, bezogen auf 1 mol der Verbindungen der allgemeinen Formel (XI), eingesetzt.

Als Hilfsmittel eignen sich die üblichen Reagenzien wie Phosgen, Carbonyldiimidazol oder Kohlensäurediethylester oder Chlorameisensäuretrichlormethylester. Bevorzugt sind Carbonyldiimidazol, Kohlensäurediethylester oder Chlorameisensäuretrichlormethylester.

Als Lösemittel eignen sich die oben aufgeführten Halogenkohlenwasserstoffe. Bevorzugt ist Methylenchlorid.

Die Verbindungen der allgemeinen Formel (VIII) sind bekannt oder können nach üblichen Methoden hergestellt werden.

Die Verbindungen der allgemeinen Formel (XII) sind größtenteils neu und können beispielsweise wie oben beschrieben hergestellt werden.

Die Verbindungen der allgemeinen Formel (VII) sind teilweise bekannt oder neu und können dann beispielsweise hergestellt werden, indem man die entsprechenden Amine mit Chlorameisensäuretrichlorethylester in einem der oben aufgeführten Lösemittel, vorzugsweise Xylol bei Rückflußtemperatur umsetzt.

Die Verbindungen der allgemeinen Formel (VIII) sind teilweise bekannt oder neu und können dann beispielsweise hergestellt werden, indem man ausgehend von den entsprechenden Carbonsäuren entweder mit Chlorameisensäureisobutylester / Aceton, Natriumazid/Wasser oder mit Diphenylphosphorylazid / Tetrahydrofuran oder mit Xylol oder Methylenchlorid in Gegenwart einer der oben angegebenen Basen, vorzugsweise Triethylamin, bei -10°C bis Raumtemperatur umsetzt.

Die Verbindungen der allgemeinen Formeln (X) und (Xa) sind teilweise bekannt oder neu und können entweder durch Abspaltung von Stickstoff aus den entsprechenden Carbonsäureaziden und Umsetzung mit den entsprechenden Alkoholen oder durch Umsetzung der entsprechenden Amine mit Chlorameisensäureestern, vorzugsweise Chlorameisensäurebenzylester in einem der oben aufgeführten Lösemittel, vorzugsweise Tetrahydrofuran oder Dioxan, in einem Temperaturbereich von -10°C bis 200°C, vorzugsweise von 0°C bis 150°C, hergestellt werden.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

| **MHK-Werte (µg/ml):** | | | | | | | |
|---|---|---|---|---|---|---|---|
| **Bsp. -Nr.** | **Staph. 133** | **Staph. 48N** | **Staph 25701** | **Staph. 9TV** | **E. coli Neumann** | **Klebs. 57 USA** | **Psdm. Bonn** |
| 10 | 4 | 4 | 4 | 2 | >64 | >64 | >64 |
| 13 | 2 | 2 | 2 | 2 | >64 | >64 | >64 |
| 14 | 2 | 2 | 2 | 1 | >64 | >64 | >64 |
| 17 | 4 | 4 | 2 | 4 | >64 | >64 | >64 |
| 18 | 2 | 4 | 4 | 1 | >64 | >64 | >64 |
| 20 | 2 | 2 | 2 | 2 | >64 | >64 | >64 |
| 25 | 2 | 2 | 2 | 1 | >64 | >64 | / |

Für schnellwachsende Mykobakterien wurde die MHK-Bestimmung in Anlehnung an die von Swenson beschriebene Methode der Bouillon Mikrodilution durchgeführt [vgl. J.M. Swenson, C. Thornberry, U.A. Silcox, Rapidly growing mycobacteria. Testing of susceptibility to 34 antimicrobial agents by broth microdilution. Antimicrobial Agents and Chemotherapy Vol, 22, 186-192 (1982)]. Abweichend davon war das mit 0,1 Vol.% Tween 80 versetzte Hirn-Herzextrakt Medium.

Die verwendeten Mykobakterienstämme wurden von der DSM (Dt. Sammlung von Mikroorganismen, Braunschweig) bezogen. Sie wurden in einer feuchten Kammer bei 37°C bebrütet.

Die MHK-Werte wurden nach 2-4 Tagen abgelesen, wenn die präparatfreien Kontrollen durch Wachstum trüb waren. Der MHK-Wert definiert sich als die niedrigste Präparatkonzentration, die makroskopisch sichtbares Wachstum völlig inhibiert.

| **MHK-Werte: Mycobacterium smegmatis** | | |
|---|---|---|
| **Keim:** | **DSM 43061** | **DSM 43078** |
| Inoculum [/ml] | 2,20E+04 | 4,20E+04 |

| Bsp.-Nr. | | |
|---|---|---|
| 14 | 4 | 4 |
| 17 | 4 | 4 |
| Isoniazid | 4 | 2 |
| Streptomycin | 4 | 4 |

### MHK-Bestimmung mit Mycoplasma pneumoniae

Mycoplasma pneumoniae Stamm PI 1428 wurde unter aeroben Bedingungen in PPLO-Medium, dem 1% Glukose, 2,5% Hefeextrakt, 20% Pferdeserum (donor horse serum) und 0,002% Phenolrot zugegeben wurde gezüchtet. MHK-Bestimmungen wurden in Anlehnung an die von ter Laak u. Mitarbeitern beschriebene Methode der Reihenmikrodilution in Flüßigmedium durchgeführt (E. A. ter Laak, A. Pijpers, J.H. Noordergraaf, E. Schoevers, J.H.M. Verheijden: Comparison of Methods for in vitro Testing of Susceptibility of Porcine Mycoplasma Species to Antimicrobial Agents; Antimicrobial Agents and Chemotherapy, Vol. 35, 228-233 (1991)). Zum Zeitpunkt des beginnenden Farbumschlags des Mediums der präparatfreien Kontrolle von rot nach gelb wurden 10 Vol% Alamar Blau zugegeben. Die Inkubation bei 37°C wurde für ca. 10 Stunden fortgesetzt und die MHK als der Wert definiert, bei dem das Medium mit der kleinsten Präparatkonzentration unverändert blau blieb.

| **Bsp.-Nr.** | **MHK (µg / ml)** |
|---|---|
| 13 | 8 |
| 14 | 8 |

Die erfindungsgemäßen Verbindungen der allgemeinen Formel (I) weisen bei geringer Toxizität ein breites antibakterielles Spektrum, speziell gegen gram-positive Bakterien sowie Mycobacterien, Haemophilus influenzae, anaerobe Keime für schnellwachsende Mykobakterien auf. Diese Eigenschaften ermöglichen ihre Verwendung als chemotherapeutische Wirkstoffe in der Human- und Tiermedizin.

Besonders wirksam sind die erfindungsgemäßen Verbindungen gegen Bakterien und bakterienähnliche Mikroorganismen, wie Mycoplasmen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die erfindungsgemäßen Verbindungen können zum Zweck der Erweiterung des Wirkungsspektrums und um eine Wirkungssteigerung zu erreichen auch mit anderen Antibiotika kombiniert werden.

### Anhang zum experimentellen Teil

### Liste der verwendeten Laufmittelgemische zur Chromatographie:

- I: Dichlormethan : Methanol
- II: Toluol : Ethylacetat
- III: Acetonitril : Wasser
- IV: Ethylacetat
- V: Petrolether : Ethylacetat
- VI: CH₂Cl₂:MeOH:NH_{3(aq)}
- VII: CH₂Cl₂ : MeOH

### Abkürzungen:

- Z: Benzyloxycarbonyl
- Boc: tert.Butoxycarbonyl
- DMF: Dimethylformamid
- Ph: Phenyl
- Me: Methyl
- THF: Tetrahydrofuran
- CDI: Carbonyldiimidazol
- DCE: Dichlorethan

### Ausgangsverbindungen

### Beispiel I

### 5-Brom-2-isocyanato-pyridin Hydrochlorid

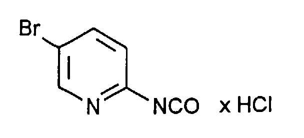

Zu einer gerührten Lösung von 100 g (0,58 mol) 2-Amino-5-brompyridin in 400 ml 1,2-Dichlorethan tropft man in der Siedehitze 78,0 ml (0,64 mol) Chlorameisensäuretrichlorethylester. Nach der Zugabe wird 2h im Rückfluß gekocht, dann darf sich das Gemisch auf Raumtemperatur abkühlen. Der entstandene Niederschlag wird durch Filtration abgetrennt, mit 100 ml 1,2-Dichlorethan gut gewaschen und im Hochvakuum über Natriumhydroxid getrocknet. Man erhält 98,3 g (72%) der Titelverbindung als gelben Feststoff.
Schmp.: 248-254°C (Zers.)
R_{f} = 0,23 (Ethylacetat)
MS (EI) m/z = 198 (M)⁺

### Beispiel II

### (5R)-3-(5-Brom-pyridin-2-yl)-5-butyryloxy-methyl-oxazolidin-2-on

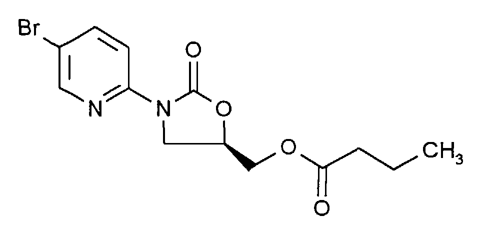

Eine Suspension von 2,17 g (25 mmol) Lithiumbromid und 5,46 g (25 mmol) Tributylphosphinoxid in 73 ml Xylol wird 1 h am Wasserabscheider gekocht. Dazu wird in der Siedehitze ein Gemisch von 58,5 ml (0,42 mol) Triethylamin und 66,6 g (0,42mol) (R)-Glycidylbutyrat getropft. Gleichzeitig werden innerhalb von 20 min 98,2 g (0,42 mol) der Verbindung aus Beispiel I portionsweise zugegeben. Nach beendeter Zugabe wird noch 1 h unter Rückfluß nachgerührt. Man läßt auf Raumtemperatur abkühlen und dampft das Lösemittel im Vakuum ab. Nach Chromatographie des Rückstands an 1 kg Kieselgel (Toluol : Ethylacetat 95:5) erhält man 37,9 g (26%) der Titelverbindung als Öl.
R_{f} = 0,43 (Toluol : Ethylacetat 4:1)
MS (FAB) m/z = 343 (M+H)⁺
¹H-NMR (250 MHz, D₆-DMSO): δ = 0,81 (t, J = 7 Hz, 3H, CH₃CH₂); 1,5 (m, 2H, CH₃CH₂CH₂CO); 2,29 (t, J = 7 Hz, 2H, CH₃CH₂CH₂CO); 3,91 (dd, J = 7 Hz, 10 Hz, 1H, H-4 trans); 4,25 (dd, J = 9 Hz, 10 Hz, 1H, H-4 cis); 4,36 (m, 2H, CH₂O); 4,97 (m, 1H, H-5); 8,08 (d, J = 1 Hz, 2H, Pyridyl H-3,4); 8,50 (d, J = 1 Hz, pyridyl H-6).

### Beispiel III

### (5R)-3-(5-Brom-pyridin-2-yl)-5-hydroxymethyl-oxazolidin-2-on

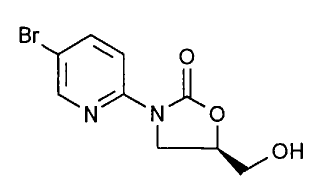

Eine Lösung von 19,6 g (57,3 mmol) der Verbindung aus Beispiel I in 125 ml wasserfreiem Methanol wird mit 185 mg (0,57 mmol) Caesiumcarbonat versetzt und 5 h bei Raumtemperatur gerührt. Das Lösemittel wird im Vakuum abgedampft und der Rückstand wird mit 30 ml Ether verrührt. Der Niederschlag wird durch Filtration abgetrennt, mit 25 ml Wasser und 5 ml Ether gewaschen und im Hochvakuum getrocknet. Man erhält 10,73 g (69%) der Titelverbindung als helle Kristalle.
Schmp.: 0,09 (Toluol : Ethylacetat 4:1)
MS (DCI, NH₃) m/z = 273 (M+H)⁺
¹H-NMR (200 MHz, CD₃OD) δ = 3,68 (d, J = 5,9 Hz, 1 H, CH₂O); 3,87 (dd, J = 4, 9 Hz, 1H, CH₂O); 4,06 (dd, J = 7, 10 Hz, 1H, H-4 trans); 4,26 (dd, J = 9, 10 Hz, 1H, H-4 cis); 4,75 (m, 1H, H-5); 7,92 (dd, J = 1,5 Hz, 10 Hz, 1H, Pyridyl H-3); 8,12 (d, J = 10 Hz, 1H, Pyridyl H-4); 8,40 (d, J = 1,5 Hz, 1H, Pyridyl H-6).

### Beispiel IV

### 5-(2-Pyridyl)-thiophen-2-carbonsäureazid

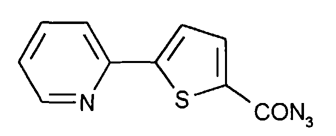

20 g (97,45 mmol) 5-(2-Pyridyl)-thiophen-2-carbonsäure werden in 200 ml Aceton gelöst, mit 15,94 ml (115 mmol) Triethylamin versetzt und auf 0°C gekühlt. Zu der so erhaltenen Reaktionslösung tropft man langsam unter Rühren eine Lösung von 14,85 ml (115 mmol) Chlorameisensäureisobutylester in 88 ml Aceton. Nach 1 h bei 0°C tropft man eine Lösung von 9,5 g (146 mmol) Natriumazid in 44 ml Wasser zu, rührt 1 h bei 0°C nach und läßt auf Raumtemperatur kommen. Die Reaktionsmischung wird auf Eiswasser gekippt und abgesaugt und so weiter umgesetzt.
Ausbeute: 21 g wasserfeuchtes Pulver.

### Beispiel V

### 5-(2-Pyridyl)-butyloxycarbonylamino-thiophen

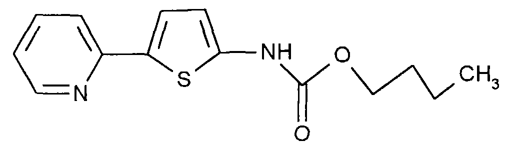

21 g der Verbindung aus Beispiel IV werden portionsweise in 400 ml siedendes n-Butanol eingetragen. Nach beendeter Gasentwicklung wird 15 min unter Rückfluß nachgerührt. Nach Abkühlen auf Raumtemperatur wird eingeengt, der Rückstand mit Ether verrührt, abgesaugt und bei 50°C im Umlufttrockenschrank getrocknet.
Ausbeute: 18,8 g (75% d.Th.)
¹H-NMR (200 MHz, D₆-DMSO): δ = 10,8 (s, 1H); 8,45 (d, J = 5 Hz, 1H); 7,68 - 7,85 (m, 2H); 7,5 (d, J = 5 Hz, 1H); 7,1 - 7,2 (m, 1H); 6,57 (d, J = 5 Hz, 1H); 4,14 (t, J = 7 Hz, 2H); 1,62 (q, J = 7 Hz, 2H); 1,39 (h, J = 7 Hz, 2H); 0,92 (t, J = 7 Hz, 3H).

### Beispiel VI

### (5R)-3-(5-(2-Pyridyl)-thien-2-yl)-5-hydroxymethyl-oxazolidin-2-on

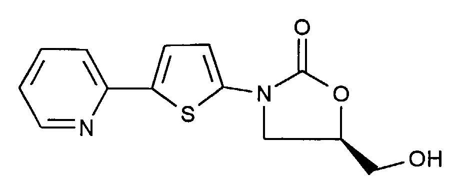

18,8 g (68 mmol) der Verbindung aus Beispiel V werden in 190 ml absolutem THF gelöst, mit 10 mg 1,10-Phenanthrolin-Hydrat versetzt und auf -70°C gekühlt. Nun werden langsam ca. 27 ml 2,5 N n-Butyllithium-Lösung in Hexan bis zum Farbumschlag nach rot zugetropft. Anschließend werden 9,6 ml (68 mmol) (R)-Glycidylbutyrat zugetropft. Man läßt auf Raumtemperatur kommen, versetzt mit gesättigter Ammoniumchloridlösung, trennt die organische Phase ab und extrahiert die wäßrige Phase zweimal mit Methylenchlorid. Die vereinigten organischen Phasen werden getrocknet (Na₂SO₄) und eingeengt. Der Rückstand wird mit Ether verrührt und abgesaugt.
Ausbeute: 15,3 g (81,5% d.Th.)
R_{f} = 0,06 (CH₂Cl₂ : CH₃OH = 100:3)
Smp.: 191°C
¹H-NMR (200 MHz, D₆-DMSO): δ = 8,45 (d, J = 5 Hz, 1H); 7,7 - 7,9 (m, 2H); 7,6 (d, J = 5 Hz, 1H); 7,15 - 7,25 (m, 1H); 6,58 (d, J = 5 Hz, 1H); 5,28 (t, J = 7 Hz, 1H); 4,77 - 4,9 (m, 1H); 4,13 (dd, J = 10 Hz, 9 Hz, 1H); 3,86 (dd, J = 10 Hz, 6 Hz, 1H); 3,55 - 3,78 (m, 2H).

Analog den Vorschriften der Beispiele I bis VI werden die in Tabelle I aufgeführten Verbindungen dargestellt:

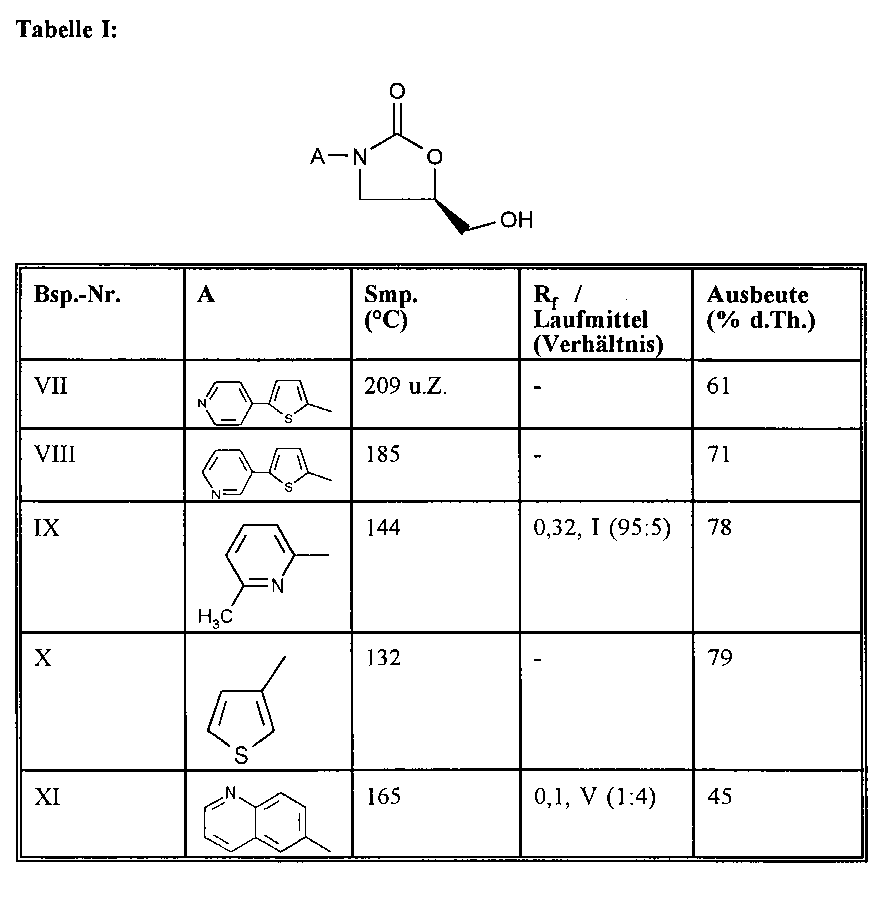

### Beispiel XII

### (5R)-3-(5-Brom-pyridin-2-yl)-5-methansulfonyloxy-methyl-oxazolidin-2-on

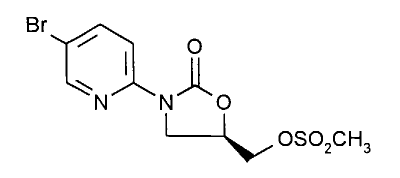

Eine auf 0°C gekühlte, gerührte Lösung von 10,5 g (38,44 mmol) der Verbindung aus Beispiel III und 6,40 ml (46,14 mmol) Triethylamin in 36 ml wasserfreiem Dichlormethan wird langsam mit 3,27 ml (42,28 mmol) Methansulfonsäurechlorid versetzt Man rührt 10 min. bei 0-5°C nach und rührt das Gemisch in 50 ml Eiswasser ein. Die organische Phase wird abgetrennt, mit 20 ml gesättigter NaHCO₃-Lösung und 20 ml Eiswasser gewaschen und über MgSO₄ getrocknet. Das Lösemittel wird im Vakuum eingedampft und der Rückstand mit 50 ml Ether verrührt, abgesaugt und im Hochvakuum getrocknet. Man erhält 12,8 g (95%) der Titelverbindung als farblose Kristalle.
Schmp.: 138-138,5°C
R_{f} = 0,65 (Dichlormethan : Methanol 95:5)
MS (DCI, NH₃) m/z = 351 (M+H)⁺
¹H-NMR (250 MHz, D₆-DMSO) δ = 3,25 (s, 3H, OSO₂CH₃); 3,91 (dd, J = 7, 10 Hz, 1H, H-4 trans); 4,27 (dd, J = 10, 10 Hz, 1H, H-4 cis); 4,52 (m, 2H, CH ₂O); 5,02 (m, 1H, H-5); 8,09 (s, 2H, Pyridyl H-3,4); 8,52 (s, 1H, Pyridyl H-6).

### Beispiel XIII

### (5R)-3-(5-Brom-pyridin-2-yl)-5-azidomethyl-oxazolidin-2-on

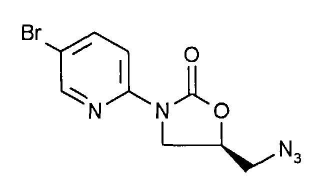

Eine gerührte Lösung von 12,5 g (35,6 mmol) der Verbindung aus Beispiel XII in 48 ml wasserfreiem DMF wird mit 3,01 g (46,28 mmol) Natriumazid versetzt und 3 h bei 70°C gerührt. Man läßt auf Raumtemperatur abkühlen und rührt in 100 ml Eiswasser ein. Der entstandene Niederschlag wird durch Filtration abgetrennt, mit 50 ml Wasser und 20 ml Petrolether gewaschen und an der Luft getrocknet. Man erhält 10,1 g (95%) der Titelverbindung als helle Kristalle.
Schmp.: 64-67°C
R_{f} = 0,63 (Toluol : Ethylacetat 2:3)
MS (DCI, NH₃) m/z = 298 (M+H)⁺
¹H-NMR (250 MHz, D₆-DMSO) δ = 3,73 (m 2H, CH₂N₃); 3,87 (dd, J = 6, 8 Hz, 1H, H-4 trans); 4,22 (dd, J = 8, 8 Hz, 1H, H-4 cis); 4,92 (m, 1H, H-5); 8,08 (s, 2H, Pyridyl H-3,4); 8,51 (s, 1H, Pyridyl H-6).

### Beispiel XIV

### (5S)-3-(5-Brom-pyridin-2-yl)-5-aminomethyl-oxazolidin-2-on Hydrochlorid

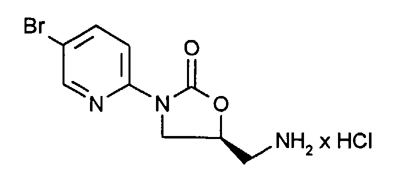

Eine gerührte Lösung von 10,1 g (33,9 mmol) der Verbindung aus Beispiel XIII in 16,5 ml 1,2-Dimethoxyethan wird auf 50°C erwärmt. Man tropft langsam 4,68 ml (4,70 mmol) Trimethylphosphit zu (Gasentwicklung) und rührt nach beendeter Zugabe noch 2 h bei 90°C nach. Nun tropft man 6,6 ml 6 N HCl zu und rührt nochmals 2 h bei 90°C nach. Man läßt auf Raumtemperatur abkühlen, trennt den Niederschlag durch Filtration ab, wäscht mit 2 x 10 ml 1,2-Dimethoxyethan und trocknet im Hochvakuum über NaOH. Man erhält 8,9 g (85%) der Titelverbindung als farblose Kristalle.
Schmp.: 260-262°C
R_{f} = 0,53 (Acetonitril : Wasser 4:1)
MS (EI) m/z = 271 (M⁺)
¹H-NMR (250 MHz, D₆-DMSO) δ = 3,28 (m, 2H, CH₂NH₂); 3,93 (dd, J = 7, 9 Hz, 1H, H-4 trans); 4,28 (dd, J = 9, 9 Hz, 1H, H-4 cis); 5,00 (m, 1H, H-5); 8,05 (s, 2H, Pyridyl H-3,4); 8,5 (m, 3H, NH₂, Pyridyl H-6).

### Beispiel XV

### (5S)-3-(5-Brom-pyridin-2-yl)-5-((tert.butyloxy)carbonyl)aminomethyl-oxazolidin-2-on

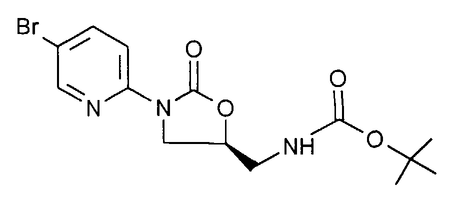

Man suspendiert 4,7 g (15 mmol) der Verbindung aus Beispiel XIV in 100 ml CH₂Cl₂. Anschließend fügt man 2,2 ml (16 mmol) Triethylamin zu, wobei eine Lösung entsteht. Man kühlt auf 0°C ab. Nun addiert man 3,5 g (16 mmol) Boc-Anhydrid so, daß die Temperatur +5°C nicht übersteigt und läßt bei Raumtemperatur über Nacht nachrühren. Man wäscht die organische Phase mit ges. NaCl-Lösung, trocknet über MgSO₄ und engt ein. Man erhält 5,4 g (97% d.Th.) des Produktes als weißen Feststoff.
Fp.: 184°C
R_{f}-Wert (Petrolether : Essigester = 10:4) = 0,30

### Beispiel XVI

### (5S)-3-(5-[3-Pyridyl]-pyridin-2-yl)-5-((tert.butyloxy)carbonyl)aminomethyloxazolidin-2-on

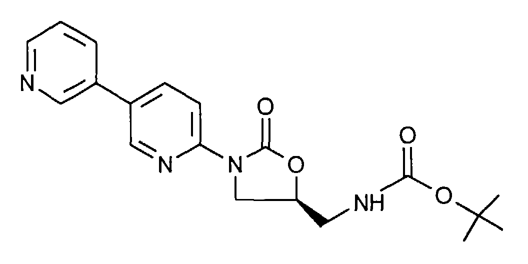

Unter Argon legt man 5,2 g (14,24 mmol) der Verbindung aus Beispiel XV und 2,81 g Diethyl-(3-pyridyl)-boran in 100 ml abs. THF vor. Man addiert die Lösung von 0,5 g (0,43 mmol) [(PPh₃)₄Pd] in 90 ml THF und 4,9 ml (9,83 mmol) 2 M Natriumcarbonatlösung. Man läßt den Ansatz 5 Tage bei Rückfluß rühren. Nach dem Abkühlen gibt man 10 g Kieselgur zu und engt ein. Der Rückstand wird auf eine mit Kieselgel gefüllte Säule aufgetragen und mit Essigester eluiert. Man erhält 4 g (76% d.Th.) der Titelverbindung
Fp.: 163°C
R_{f}-Wert = 0,36 (CH₂Cl₂ : MeOH = 100:5)

### Beispiel XVII

### (5S)-3-(5-[3-Pyridyl]-pyridin-2-yl)-5-aminomethyl-oxazolidin-2-on Trihydrochlorid

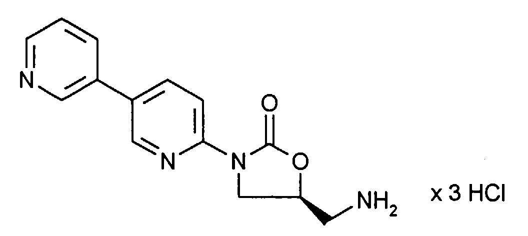

3,8 g (10,3 mmol) der Verbindung aus Beispiel XVI werden in 25 ml Dioxan suspendiert. Man addiert 32,1 ml einer 4 M HCl-Lösung in Dioxan undläßt über Nacht bei Raumtemperatur rühren. Man engt ein und rührt den Rückstand mit Ether aus. Anschließend wird der Feststoff durch eine Fritte abgesaugt und mit Ether nachgewaschen. Man trocknet am Hochvakuum und erhält 3,7 g (95% d.Th.) der Titelverbindung.
Fp.: >250°C
MS (EI): 271 (M⁺), 172
¹H-NMR (200 MHz, DMSO-d₆): δ = 9,35 (sb, 1H); 8,93 (m, 3H); 8,6 (breit, 3H); 8,42 (dd, J = 9, J = 3, 1H); 8,24 (d, J = 9, 1H); 8,11 (dd, J = 7,5, J = 6,5, 1H); 6,7 - 5,3 (breit, 2H); 5,06 (m, 1H); 4,38 (tr, J = 10, 1H); 4,03 (dd, J = 10, J = 7,5, 1H); 3,29 (m, 2H).

Analog den Vorschriften der Beispiele XII bis XVII wurden die in Tabelle II aufgeführten Verbindungen dargestellt:

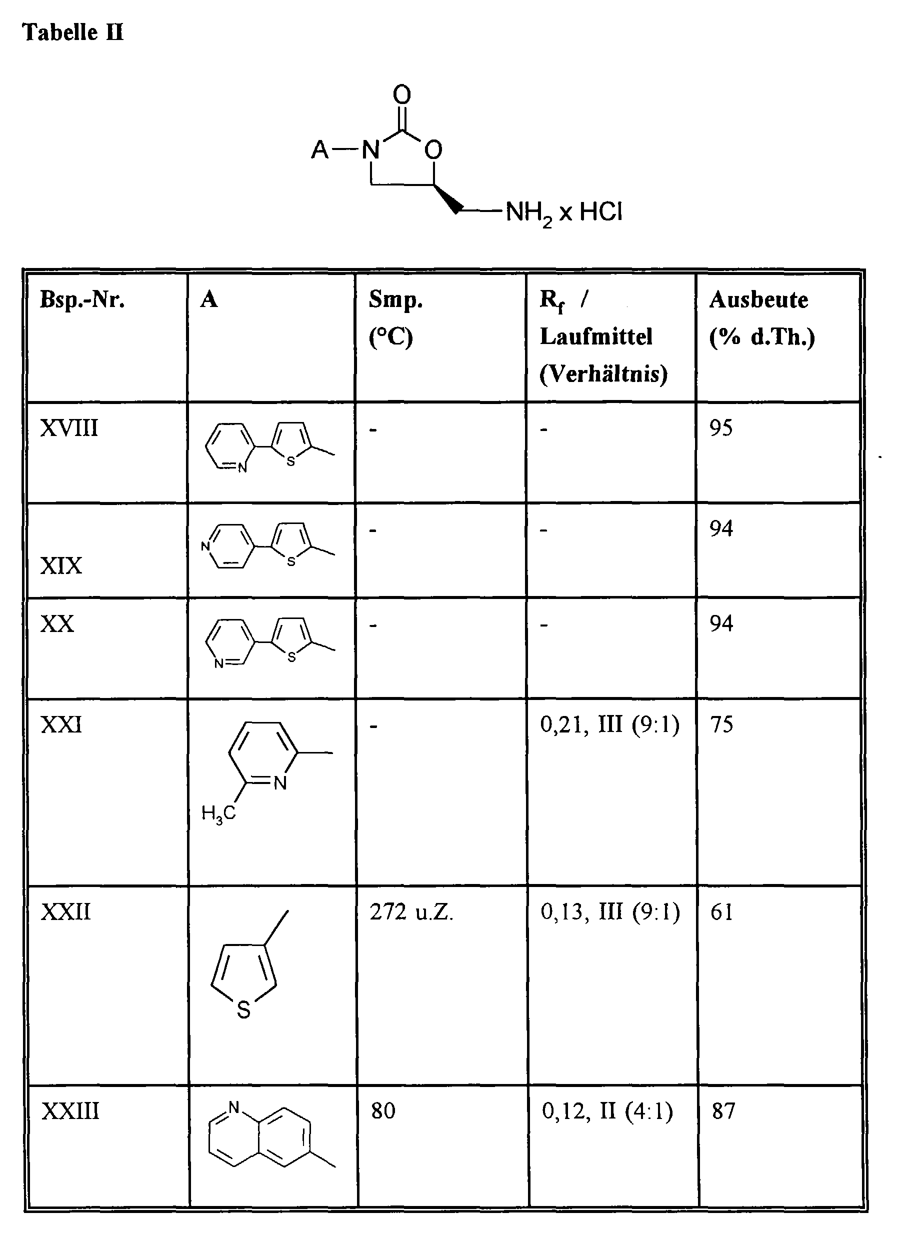

In Analogie zur Vorschrift der Beispiele I bis VI werden die in der Tabelle III aufgeführten Verbindungen hergestellt.

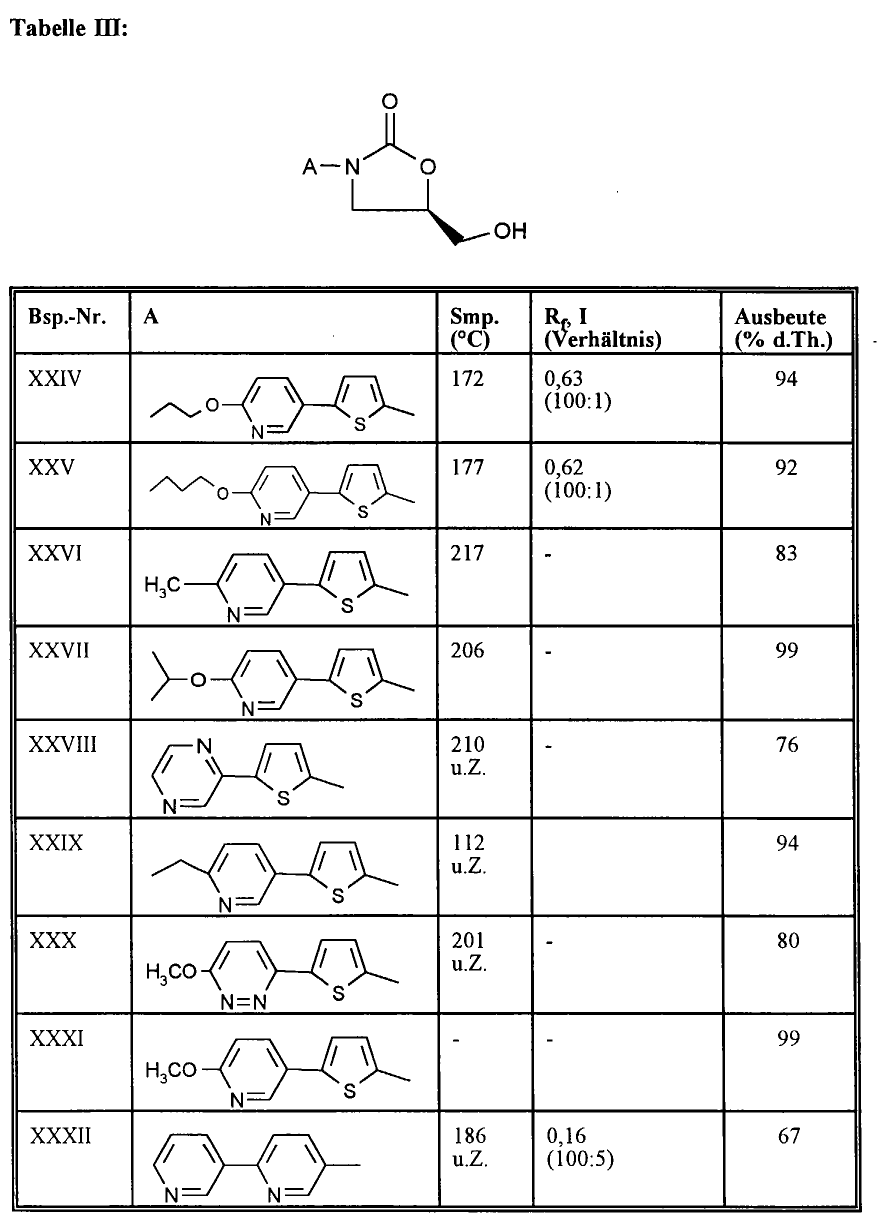

Die in der Tabelle IV aufgeführten Verbindungen werden in Analogie zu den Vorschriften des Beispiels XIV hergestellt und als freie Base nach wäßriger Aufarbeitung isoliert.

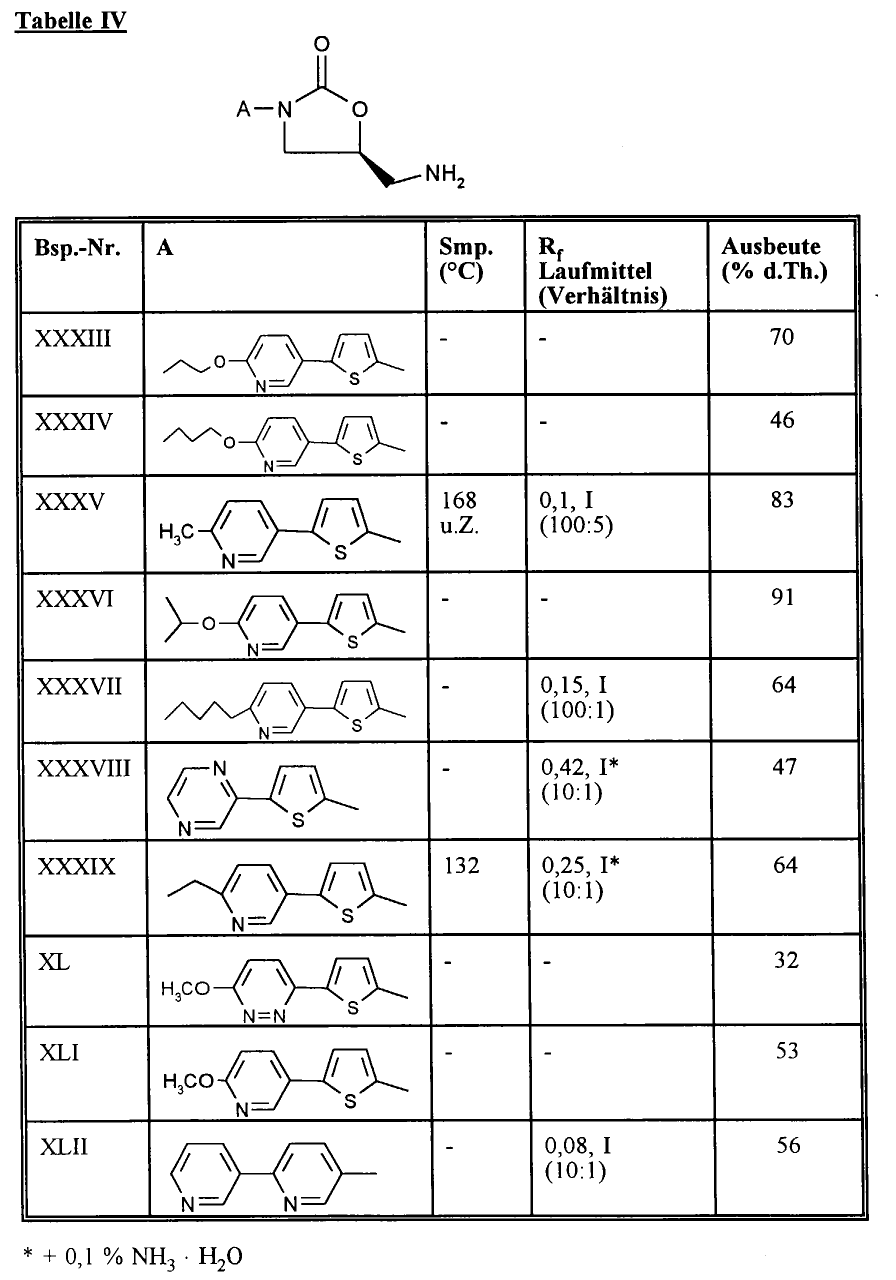

### Herstellungsbeispiele

### Beispiel 1

### (5R)-3-(5-(2-Pyridyl)thien-2-yl)-5-ethyloxycarbonyl-aminomethyl-oxazolidin-2-on

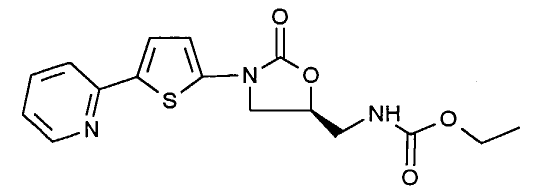

348 mg (1 mmol) der Verbindung aus Beispiel XXVIII werden mit 2 ml Methylenchlorid und 0,33 ml (2,4 mmol) Triethylamin versetzt. Die so erhaltene Reaktionsmischung wird auf 0°C gekühlt und mit 115 µl (1,2 mmol) Chlorameisensäureethylester versetzt. Man läßt über Nacht auf Raumtemperatur kommen, engt ein und chromatographiert an Kieselgel (Methylenchlorid/Methanol 100/2).
Ausbeute: 170 mg (49% d.Th.)
Smp.: 187°C u.Z.
R_{f}=0,48 (I, 100:5)
MS (EI): 348 (M+H)⁺ (100%)

Analog der Vorschrift des Beispiels 1 werden die in Tabelle 1 aufgeführten Verbindungen dargestellt:

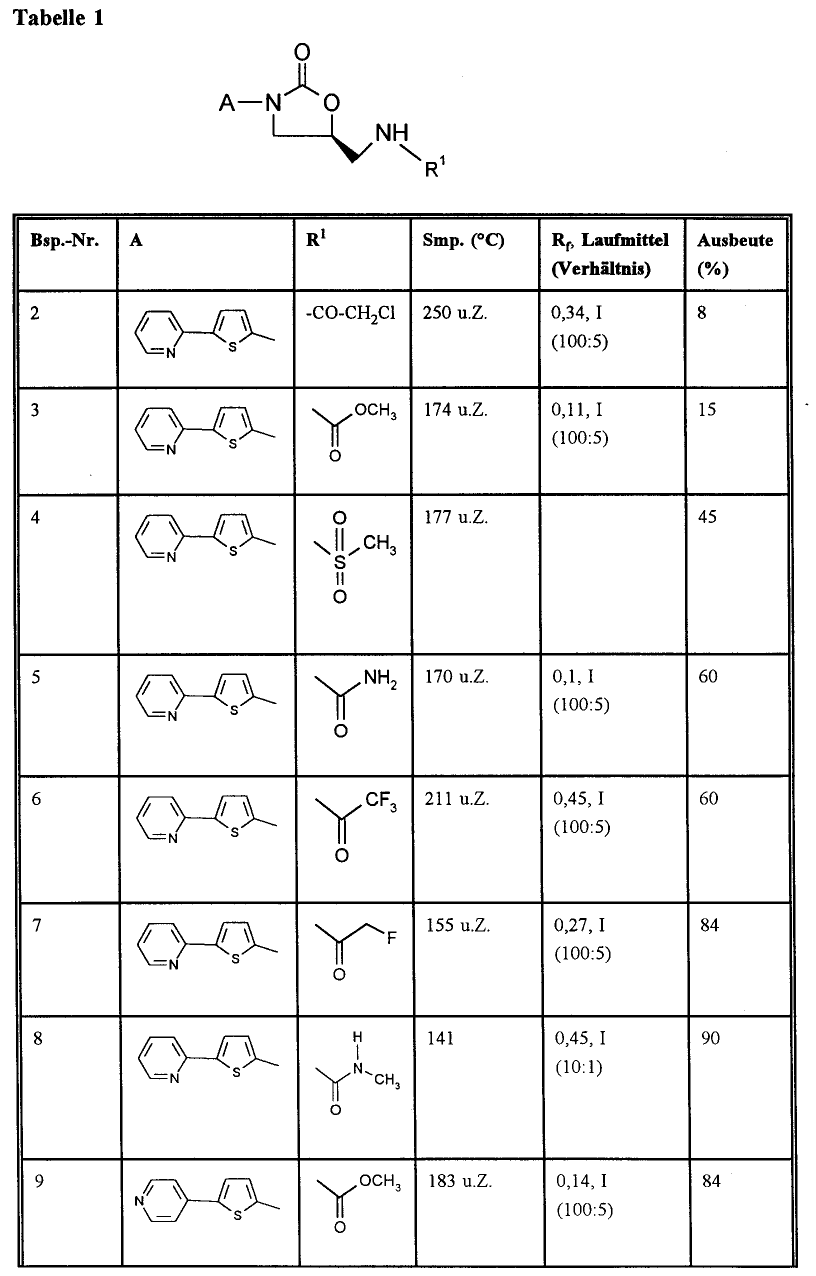

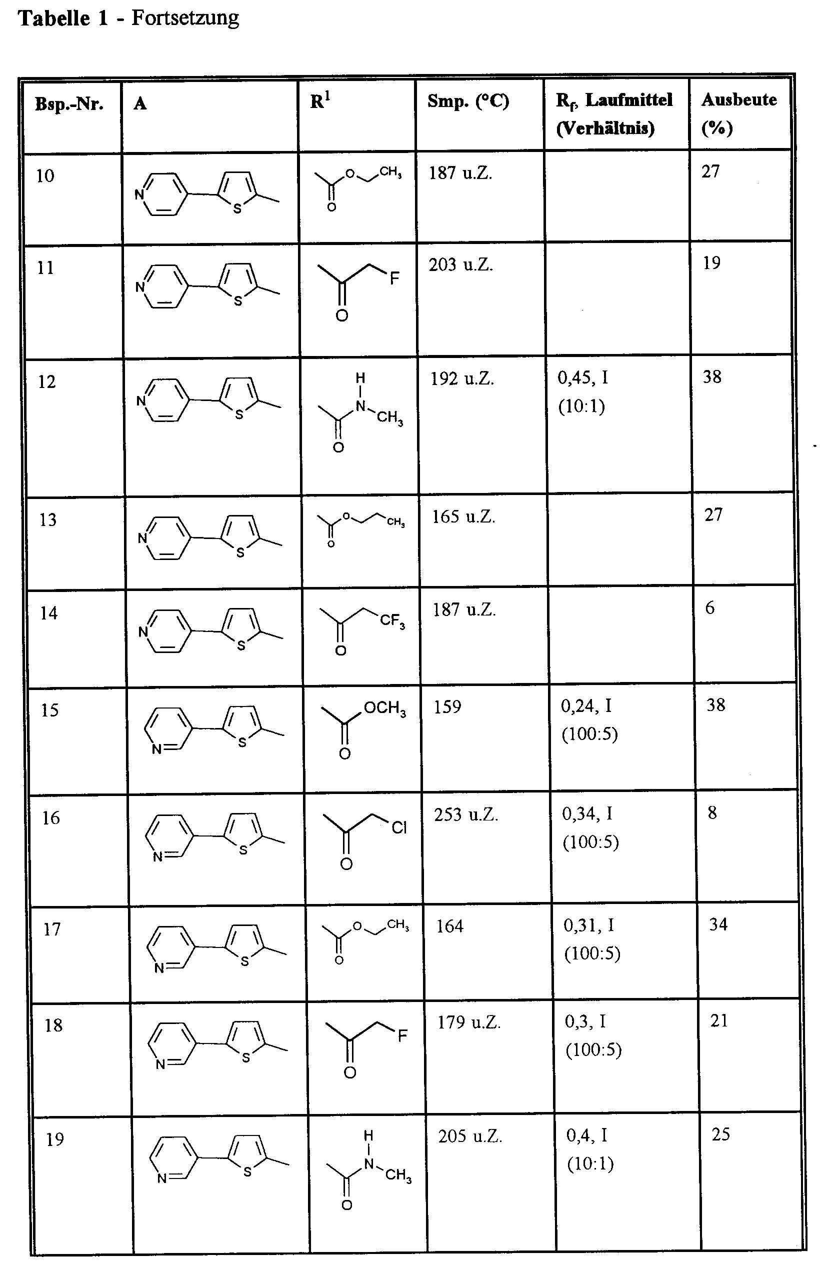

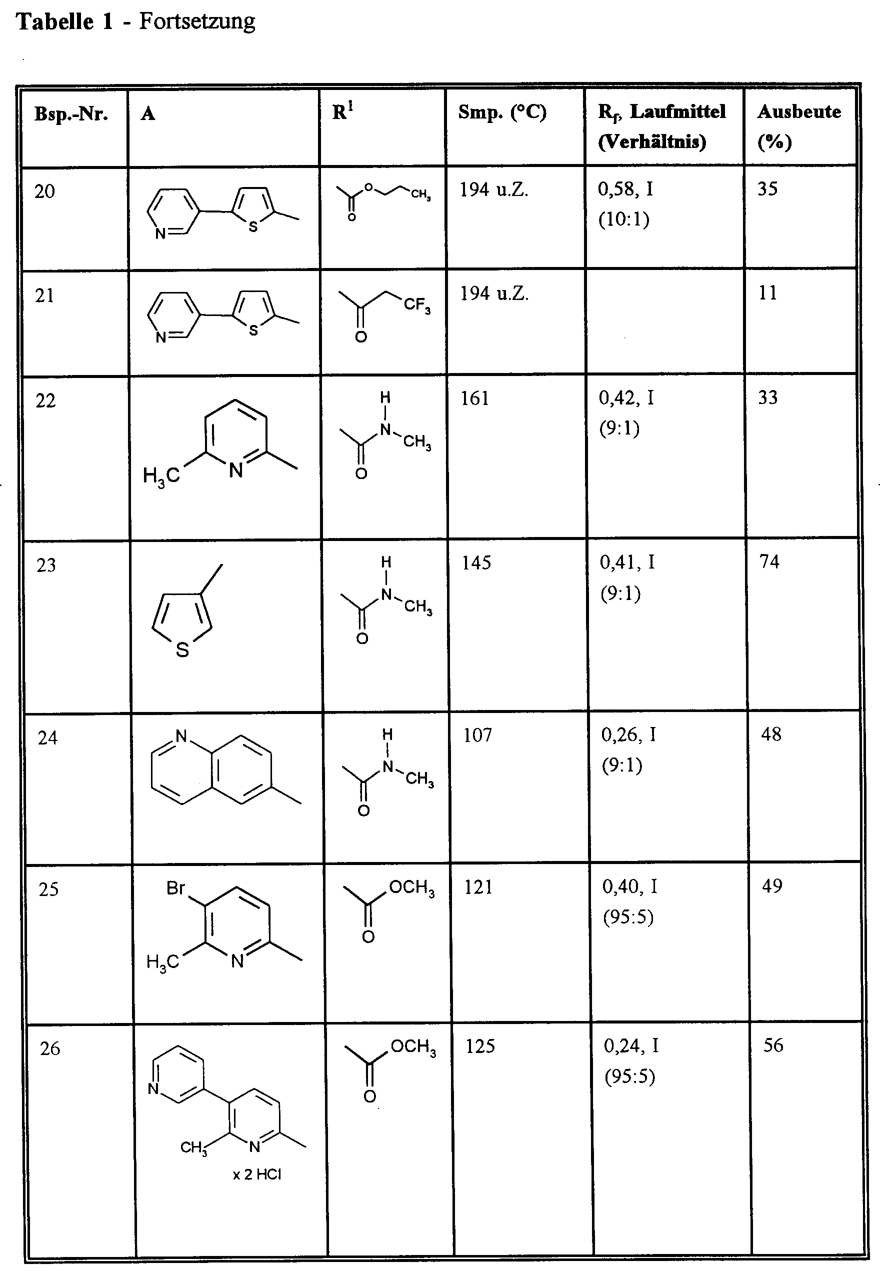

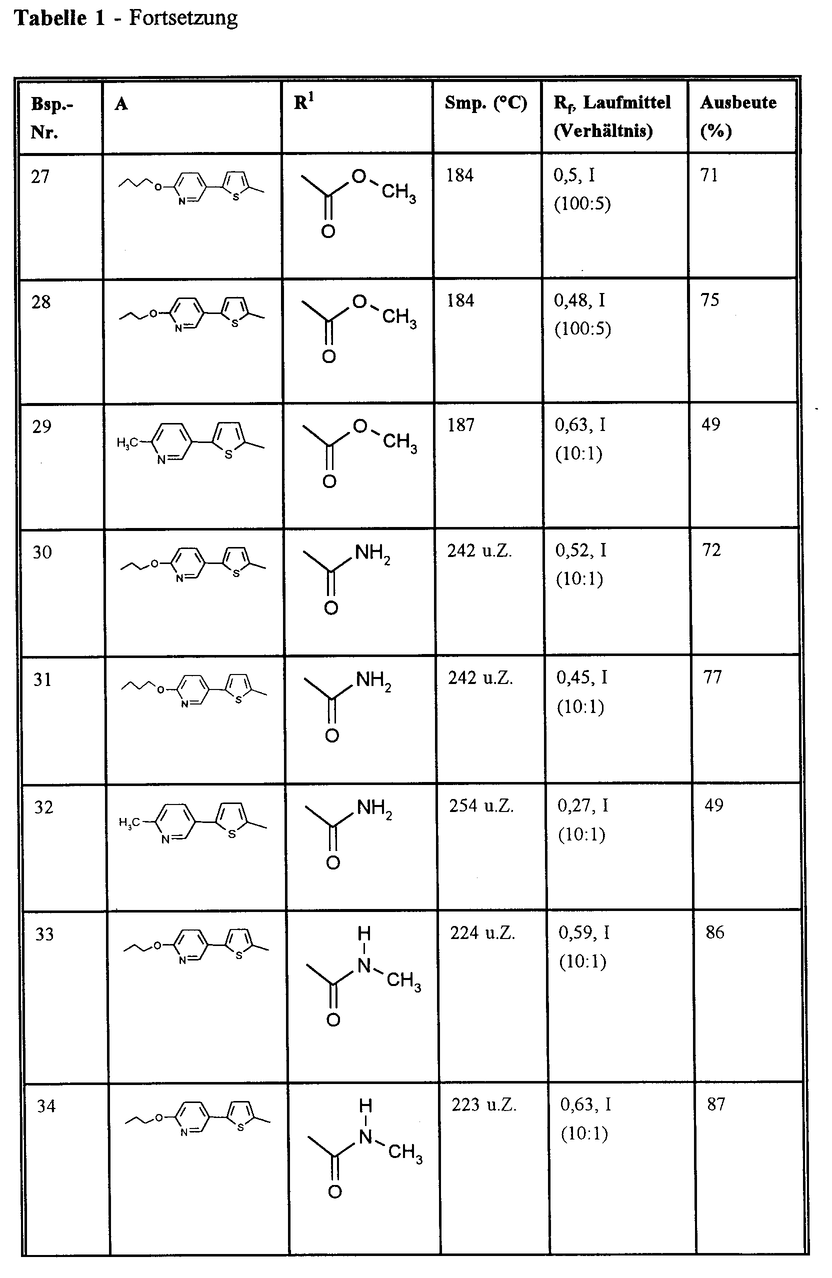

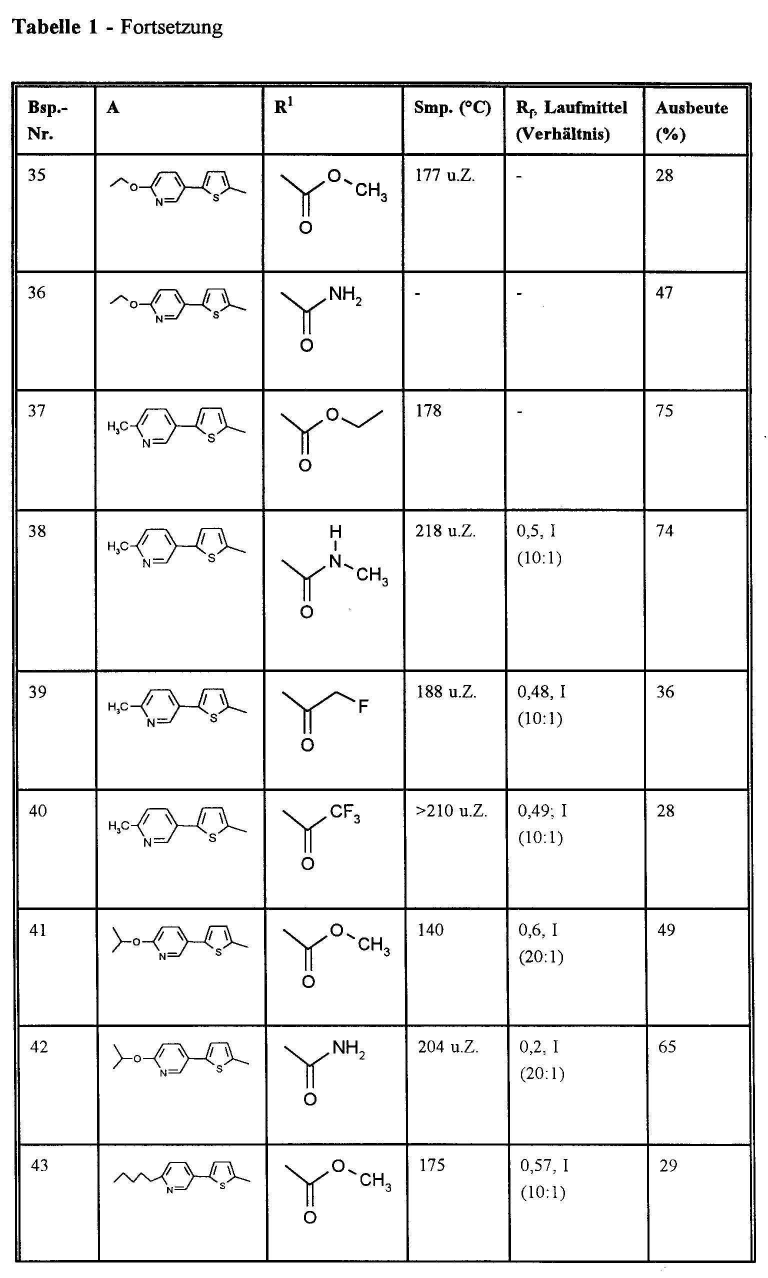

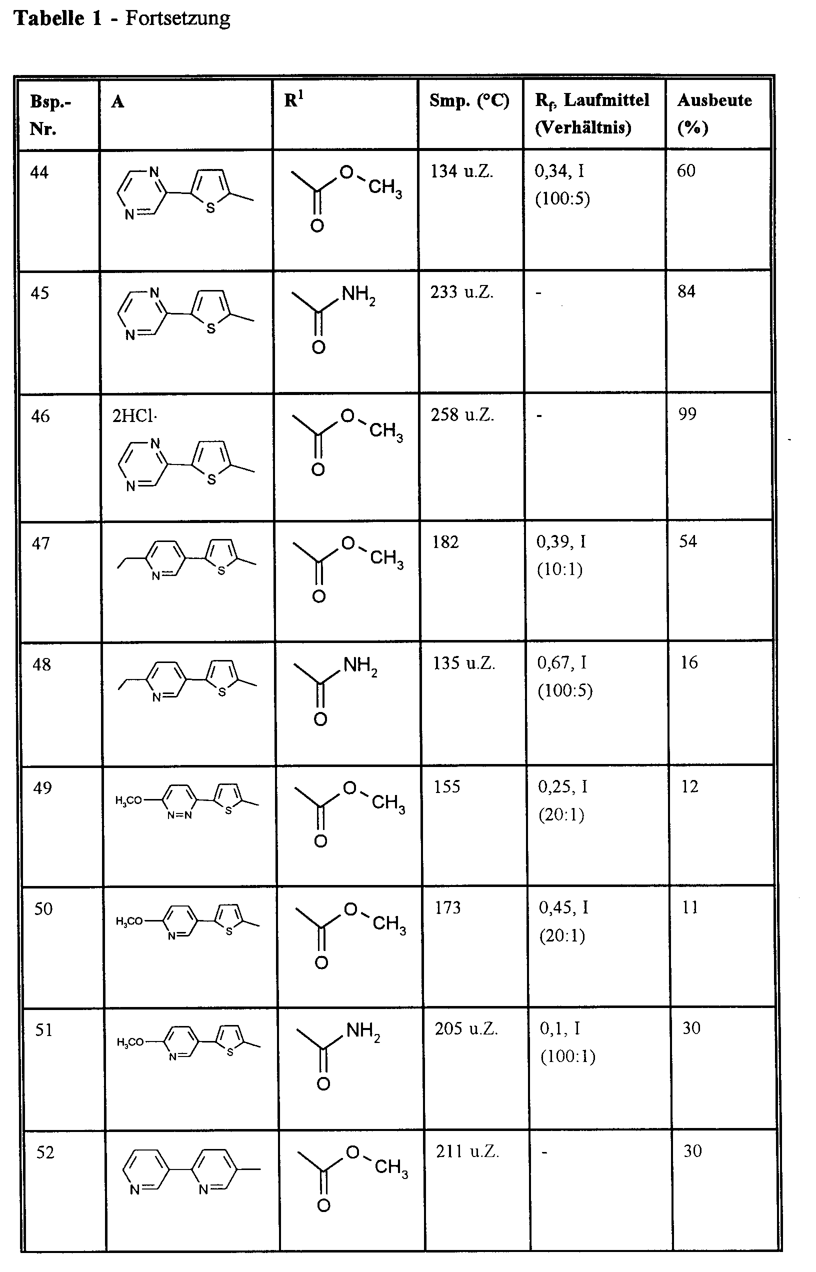

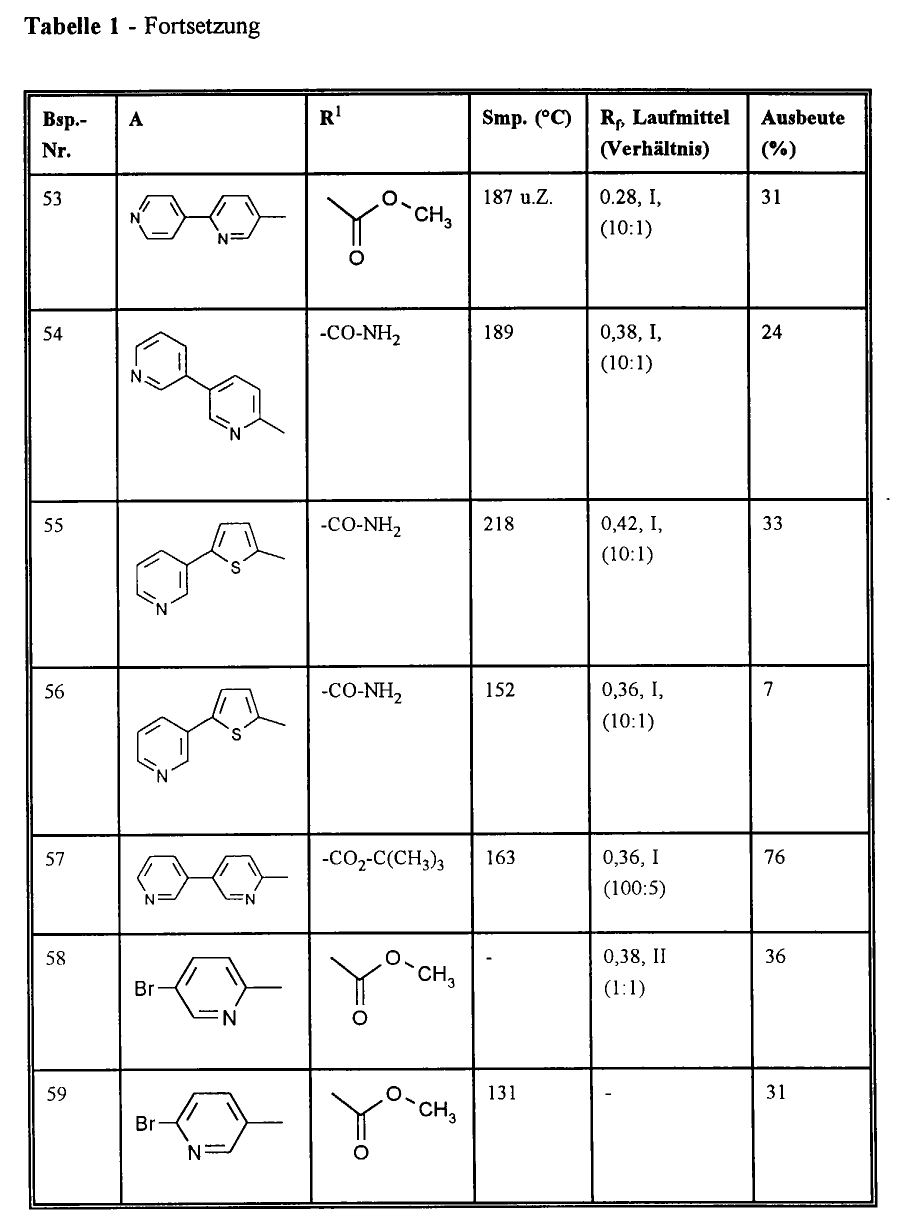

In Analogie zur Vorschrift des Beispiels XVI wurden die in der Tabelle 2 aufgeführten Verbindungen dargestellt.

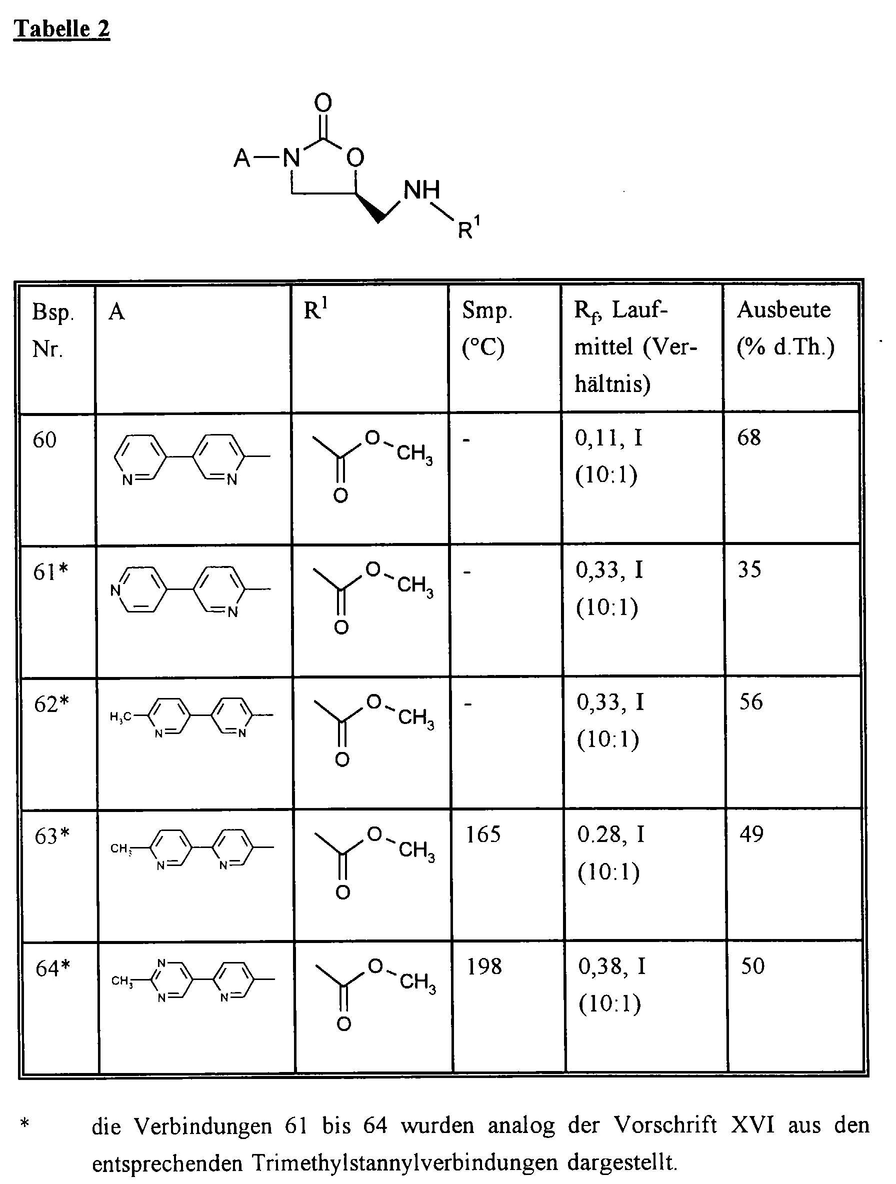

### Beispiel 65

### (5S)-3-(5-(4-Pyridyl)-pyridin-2-yl)-3-(methoxycarbonylaminomethyl)-2-oxazolidinon-Hydrochlorid

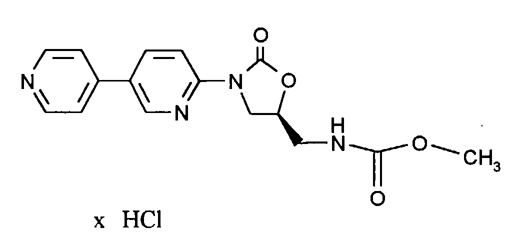

Zu einer Lösung aus 500 mg (1,6 mmol) der Verbindung aus Beispiel 61 in 60 ml Dioxan werden 2,04 ml (8.16 mmol) 4 N HCl in Dioxan und anschließend 200 ml Ether gegeben. Der ausgefallene Niederschlag wird abgesaugt, mit Ether gewaschen und im Hochvakuum getrocknet.
Ausbeute: 0.555g (90%)
¹H-NMR (200 MHz, DMSO-d₆): δ = 8.9-9.2 (m.3H), 8.45-8.55 (m.3H), 8.30 (bt, 1H, NH), 8.20 (d, 2H), 4,70 (m, 1H), 4.30 (dd, 1H), 3.95 (dd, 1H), 3.52 (s, 3H), 3.45 (m, 2H).

In Analogie zur Vorschrift des Beispiels 65 wurden die in der Tabelle 3 aufgeführten Verbindungen dargestellt.

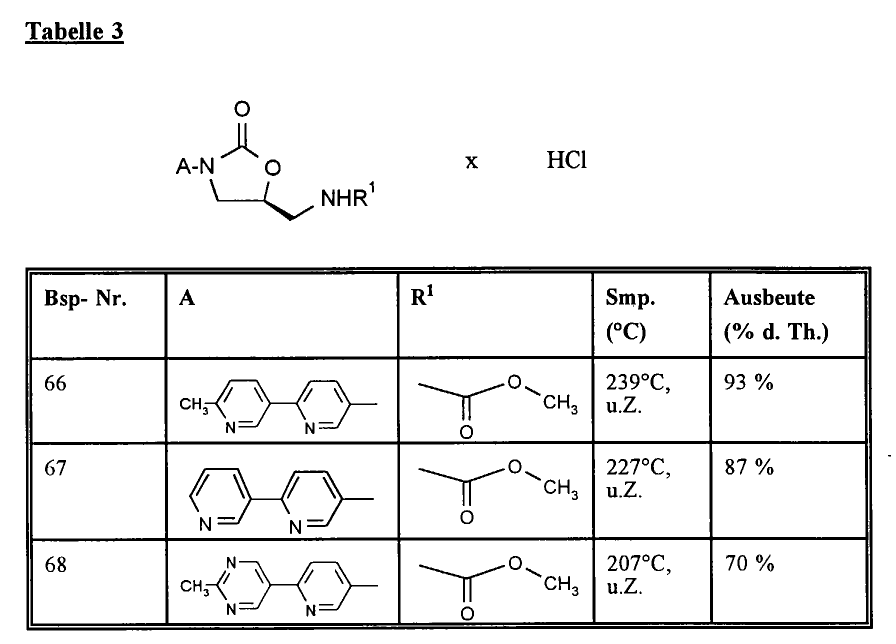

## Patentansprüche

1. Heteroaryl-Oxazolidinone der allgemeinen Formel (I) 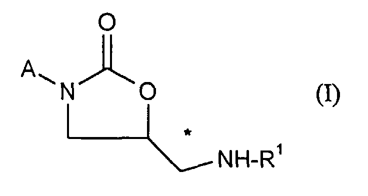 in welcher
R¹ für einen Rest der Formel D-R², -CO-R³ oder -CO-NR⁴R⁵ steht,
worin
D die CO₂- oder SO₂-Gruppe bedeutet,
R² Phenyl oder geradkettiges oder verzweigtes Alkyl mit bis zu 7 Kohlenstoffatomen bedeutet,
R³ Trifluormethyl oder geradkettiges oder verzweigtes Alkyl mit bis zu 6 Kohlenstoffatomen bedeutet, das durch Halogen oder Trifluormethyl substituiert ist,
R⁴ und R⁵ gleich oder verschieden sind und Wasserstoff, Phenyl oder geradkettiges oder verzweigtes Alkyl mit bis zu 5 Kohlenstoffatomen bedeuten,
A für einen über ein Kohlenstoffatom direkt gebundenen 6-gliedrigen, aromatischen Heterocyclus mit mindestens einem Stickstoffatom steht, oder
für einen über ein Kohlenstoffatom direkt gebundenen, jeweils 6-gliedrigen, bi- oder tricyclischen aromatischen Rest mit mindestens einem stickstoffhaltigen Ring steht, oder
für β-Carbolin-3-yl oder für über den 6-Ring direkt gebundenes Indolizinyl steht, oder
für einen über ein Kohlenstoffatom direkt gebundenen 5-gliedrigen aromatischen Heterocyclus mit bis zu 3 Heteroatomen aus der Reihe S, N und/oder O steht, der zusätzlich einen annellierten Benzol- oder Naphthylring besitzen kann, wobei alle Cyclen gegebenenfalls jeweils bis zu 3-fach gleich oder verschieden durch Carboxy, Halogen, Cyano, Mercapto, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -NR⁶R⁷ substituiert sein kann,
worin
R⁶ und R⁷ gleich oder verschieden sind und Wasserstoff, Cycloalkyl mit 3 bis 6 Kohlenstoffatomen oder geradkettiges oder verzweigtes Alkyl mit bis zu 5 Kohlenstoffatomen oder Phenyl bedeuten,
oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,
und/oder
die Cyclen gegebenenfalls durch eine Gruppe der Formel -NR⁶'R⁷' substituiert sind,
worin
R^{6'} und R^{7'} gleich oder verschieden sind und die oben angegebene Bedeutung von R⁶ und R⁷ haben und mit diesen gleich oder verschieden sind,
und/oder
die Cyclen gegebenenfalls durch (C₂-C₈)-Alkenylphenyl, Phenyl oder durch einen 5- oder 6-gliedrigen gesättigten oder ungesättigten Heterocyclus mit bis zu 3 Heteroatomen aus der Reihe S, N und/oder O substituiert sind, die ihrerseits gegebenenfalls durch eine Gruppe der Formel -CO-NR⁸R⁹, -NR¹⁰R¹¹, -NR¹²-S(O)₂-R¹³, R¹⁴R¹⁵N-SO₂- oder R¹⁶-S(O)ₐ- substituiert sind,
worin
a eine Zahl 0, 1 oder 2 bedeutet,
R⁸, R⁹, R¹², R¹⁴ und R¹⁵ gleich oder verschieden sind und Wasserstoff, geradkettiges oder verzweigtes Alkyl mit bis zu 6 Kohlenstoffatomen oder Phenyl bedeuten,
R¹⁰ und R¹¹ gleich oder verschieden sind und die oben angegebene Bedeutung von R⁶ und R⁷ haben und mit diesen gleich oder verschieden sind,
R¹³ und R¹⁶ gleich oder verschieden sind und geradkettiges oder verzweigtes Alkyl mit bis zu 4 Kohlenstoffatomen oder Phenyl bedeuten, das gegebenenfalls durch geradkettiges oder verzweigtes Alkyl mit bis zu 4 Kohlenstoffatomen substituiert ist,
und/oder ihrerseits gegebenenfalls bis zu 2-fach gleich oder verschieden durch Carboxy, Halogen, Cyano, Mercapto, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -NR¹⁷R¹⁸ substituiert sein kann,
worin
R¹⁷ und R¹⁸ die oben angegebene Bedeutung von R⁶ und R⁷ haben und mit diesen gleich oder verschieden sind,
und/oder
die Cyclen gegebenenfalls durch einen Rest der Formel 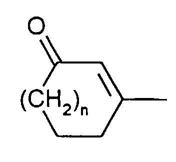 substituiert sind
worin
n eine Zahl 0, 1 oder 2 bedeutet,
und deren Stereoisomere, Stereoisomerengemische und Salze.

2. Verbindungen der allgemeinen Formel (I) gemäß Anspruch 1,
in welcher
R¹ für einen Rest der Formel D-R², -CO-R³ oder -CO-NR⁴R⁵ steht,
worin
D die CO₂- oder SO₂-Gruppe bedeutet,
R² Phenyl oder geradkettiges oder verzweigtes Alkyl mit bis zu 5 Kohlenstoffatomen bedeutet,
R³ Trifluormethyl oder geradkettiges oder verzweigtes Alkyl mit bis zu 5 Kohlenstoffatomen bedeutet, das durch Fluor, Chlor, Brom oder Trifluormethyl substituiert ist,
R⁴ und R⁵ gleich oder verschieden sind und
Wasserstoff, Phenyl oder geradkettiges oder verzweigtes Alkyl mit bis zu 4 Kohlenstoffatomen bedeuten,
A für über ein Kohlenstoffatom gebundenes Cinnolinyl, Pteridinyl, Acridinyl, Chinazolinyl, Chinoxalinyl, Naphthyridinyl, Phthalazinyl, Chinolyl, Isochinolyl, Pyridyl, Pyrazinyl, Pyrimidinyl oder Pyridazinyl steht, oder
für über ein Kohlenstoffatom direkt gebundenes Pyrrolyl, Imidazolyl, Furyl, Thienyl, Thiazolyl, Oxazolyl, Isothiazolyl, Isoxazolyl oder Furazanyl steht, oder für ebenfalls über ein Kohlenstoffatom des 5-gliedrigen Ringes direkt gebundenes Indolyl, Benzo[b]thienyl, Naphto[2,3-b]thienyl, Benzo[b]thiazolyl, Benzo[b]imidazolyl oder Benzo[b]furanyl steht, die gegebenenfalls bis zu 3-fach gleich oder verschieden durch Fluor, Chlor, Brom, geradkettiges oder verzweigtes Alkyl, Acyl oder Alkoxy mit jeweils bis zu 5 Kohlenstoffatomen oder durch Phenyl, Pyrimidyl, Pyridazinyl oder Pyridyl substituiert sind, die ihrerseits durch geradkettiges oder verzweigtes Alkyl, Alkoxy oder Acyl mit jeweils bis zu 5 Kohlenstoffatomen, oder durch eine Gruppe der Formel -NR⁶R⁷ substituiert sein können,
worin
R⁶ und R⁷ gleich oder verschieden sind und
Wasserstoff Phenyl, Cyolopropyl, Cyclobutyl, Cyclopentyl, Cyclohexyl oder geradkettiges oder verzweigtes Alkyl mit bis zu 4 Kohlenstoffatomen bedeuten,
und deren Stereoisomere, Stereoisomerengemische und Salze.

3. Verbindungen der allgemeinen Formel (I) gemäß Anspruch 1,
in welcher
R¹ für einen Rest der Formel D-R², -CO-R³ oder -CO-NR⁴R⁵ steht,
worin
D die CO₂- oder SO₂-Gruppe bedeutet,
R² Phenyl oder geradkettiges oder verzweigtes Alkyl mit bis zu 4 Kohlenstoffatomen bedeutet,
R³ Trifluormethyl oder geradkettiges oder verzweigtes Alkyl mit bis zu 3 Kohlenstoffatomen bedeutet, das durch Fluor, Chlor, Brom oder Trifluormethyl substituiert ist,
R⁴ und R⁵ gleich oder verschieden sind und
Wasserstoff, Phenyl oder geradkettiges oder verzweigtes Alkyl mit bis zu 3 Kohlenstoffatomen bedeuten,
A für Thienyl, Pyridyl oder Chinolyl steht, die gegebenenfalls bis zu 2-fach gleich oder verschieden durch Chlor, Brom, geradkettiges oder verzweigtes Alkyl oder Alkoxy mit jeweils bis zu 5 Kohlenstoffatomen oder durch Phenyl, Pyridazinyl, Pyrimidyl oder Pyridyl substituiert sind, die ihrerseits durch geradkettiges oder verzweigtes Alkyl, Alkoxy oder Acyl mit jeweils bis zu 5 Kohlenstoffatomen, oder durch eine Gruppe der Formel -NR⁶R⁷ substituiert sein können,
worin
R⁶ und R⁷ gleich oder verschieden sind und
Wasserstoff, Phenyl, Cyclopropyl, Cyclobutyl, Cyclopentyl, Cyclohexyl oder geradkettiges oder verzweigtes Alkyl mit bis zu 3 Kohlenstoffatomen bedeuten,
und deren Stereoisomere, Stereoisomerengemische und Salze.

4. Verbindungen der allgemeinen Formel (V) 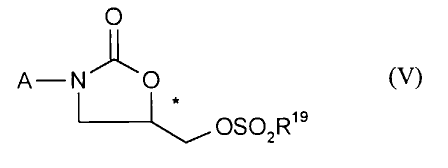 in welcher
A die in Anspruch 1 angegebene Bedeutung hat
und
R¹⁹ für C₁-C₄-Alkyl steht,
und deren Stereoisomere, Stereoisomerengemische und deren Salze.

5. Verbindungen der allgemeinen Formel (VI) 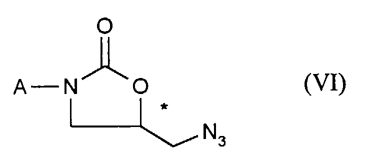 in welcher
A die in Anspruch 1 angegebene Bedeutung hat,
und deren Stereoisomere, Stereoisomerengemische und deren Salze.

6. Verfahren zur Herstellung von Verbindungen der allgemeinen Formel (I) gemäß Anspruch 1, dadurch gekennzeichnet, daß man
Verbindungen der allgemeinen Formel (II) 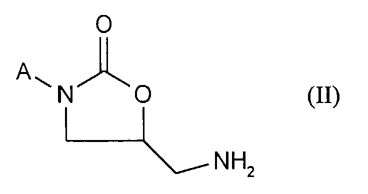 in welcher
A die in Anspruch 1 angegebene Bedeutung hat,
mit Verbindungen der allgemeinen Formel (III)
R¹-E (III)
in welcher
R¹ die in Anspruch 1 angegebene Bedeutung hat,
und
E für Halogen oder für geradkettiges oder verzweigtes Alkylthio, Alkoxy oder Oxyalkoxycarbonyl mit jeweils bis zu 5 Kohlenstoffatomen steht,
in inerten Lösemitteln, gegebenenfalls in Anwesenheit einer Base umsetzt, gegebenenfalls nach üblichen Methoden die Verbindungen in ihre Salze überführt bzw. aus ihren Salzen freisetzt, und gegebenenfalls nach üblichen Methoden die Stereoisomeren trennt.

7. Verbindungen der allgemeinen Formel (I) gemäß Anspruch 1 zur Verwendung bei der Bekämpfung von Krankheiten.

8. Verwendung von Verbindungen der allgemeinen Formel (I) gemäß Anspruch 1 zur Herstellung von Arzneimitteln.

9. Arzneimittel enthaltend Verbindungen der allgemeinen Formel (I) gemäß Anspruch 1.
